# EUROPEAN PATENT SPECIFICATION

(11) **EP 2 313 386 B1**
(45) Date of publication and mention of the grant of the patent: **04.01.2012**
(21) Application number: 09800073.0
(22) Date of filing: 23.07.2009
(51) Int. Cl.: C07D 307/33, C07C 235/02

(54) **SYNTHESIS ROUTES TO 2(S),4(S),5(S),7(S)-2,7-DIALKYL-4-HYDROXY-5-AMINO-8-ARYL-OCTANOYL AMIDES**
WEGE ZUR SYNTHESE VON 2(S),4(S),5(S),7(S)-2,7-DIALKYL-4-HYDROXY-5-AMINO-8-ARYL-OCTANOYL-AMIDEN
VOIES DE SYNTHÈSE DES AMIDES 2(S),4(S),5(S),7(S)-2,7-DIALKYL-4-HYDROXY-5-AMINO-8- ARYL-OCTANOYLE

(30) Priority: 23.07.2008 EP 08160971
(43) Date of publication of application: 27.04.2011
(73) Proprietor: DSM IP Assets B.V., 6411 TE Heerlen (NL)
(72) Inventor: VRIES DE, Andreas, Hendrikus, Maria, NL-6212 CR Maastricht (NL); VERZIJL, Gerardus, Karel, Maria, NL-5855 AR Well (NL); HERMSEN, Petrus, Johannes, NL-5961 VC Horst (NL); CASTELIJNS, Anna, Maria, Cornelia, Francisca, NL-6176 JB Spaubeek (NL)
(74) Representative: Hoogendam, Gerrie Christine
(86) International application number: PCT/EP2009/059526
(87) International publication number: WO 2010/010165

(56) References cited:
- RUEGER H ET AL: "A convergent synthesis approach towards CGP60536B, a non-peptide orally potent renin inhibitor, via an enantiomerically pure ketolactone intermediate" TETRAHEDRON LETTERS, ELSEVIER, AMSTERDAM, vol. 41, no. 51, 16 December 2000 (2000-12-16), pages 10085-10089, XP004225222 ISSN: 0040-4039 cited in the application
- CHELUCCI G ET AL: "An easy route to optically active 1-substituted-1-pyridyl-methylamines by diastereoselective reduction of enantiopure N-tert-butanesulfinyl ketimines" TETRAHEDRON ASYMMETRY, PERGAMON PRESS LTD, OXFORD, GB, vol. 17, no. 22, 27 November 2006 (2006-11-27), pages 3163-3169, XP024962299 ISSN: 0957-4166 [retrieved on 2006-11-27]
- SANDHAM D A ET AL: "A convergent synthesis of the renin inhibitor CGP60536B" TETRAHEDRON LETTERS, ELSEVIER, AMSTERDAM, vol. 41, no. 51, 16 December 2000 (2000-12-16), pages 10091-10094, XP004225223 ISSN: 0040-4039 cited in the application
- IRELAND T ET AL: "Identification of new catalysts for the asymmetric reduction of imines into chiral amines with polymethylhydrosiloxane using high-throughput screening" TETRAHEDRON LETTERS, ELSEVIER, AMSTERDAM, vol. 45, no. 22, 24 May 2004 (2004-05-24), pages 4383-4387, XP004506366 ISSN: 0040-4039
- CIMARELLI C ET AL: "Asymmetric reduction of enantiopure imines with zinc borohydride: stereoselective synthesis of chiral amines" TETRAHEDRON ASYMMETRY, PERGAMON PRESS LTD, OXFORD, GB, vol. 11, no. 12, 30 June 2000 (2000-06-30), pages 2555-2563, XP004209679 ISSN: 0957-4166
- MARX ET AL.: "Asymmetric synthesis with Chiral Hydrogenolysable Amines. omega-Imino Esters Reduction: A Diastereoselective Route to omega-Alkyl Lactames" TETRAHEDRON LETTERS, vol. 33, no. 30, 1992, pages 4307-4310, XP002549372

## Description

The invention relates to a process for the preparation of compound according to formula (13) or its ring-closed form according to formula (2), which compound is an important building block in convergent synthesis routes to 2(*S*),4(*S*),5(*S*),7(*S*)-2,7-dialkyl-4-hydroxy-5-amino-8-aryl-octanoyl amides (compounds according to formula 13 with X stands for NHR₅), or pharmaceutically acceptable salts thereof, such as the compound Aliskiren, and to a process for the preparation of these octanoyl amides, comprising reacting said building block.

From a paper by Rueger et al, Tetrahedron letters 41 (2000) p.10085-10089, it is known that a ketone containing compound according to formula (4a) 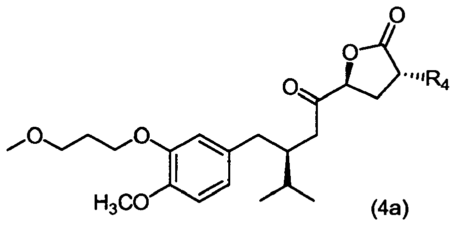 with R₄ being 2-propyl is a potentially interesting building block for use in a convergent synthesis route to Aliskiren. However, the conversion of the ketone according to formula (4a) to the corresponding enantio-enriched amine (according to formula 2), and subsequently to the compound Aliskiren or related compounds has so far remained unsatisfactory with respect to yield and/or stereoselectivity.

From a paper by Sandham et al, Tetrahedron letters 41 (2000) p.10091-10094, it is known that compounds according to formula (13) with X stands for NHR₅ are prepared by the corresponding 5(*R*) hydroxy compound using substitution chemistry with sodium azide. The corresponding 5(*R*) hydroxy compound had been prepared in a inseparable mixture of diastereomers, which only could be purified at the end stage of the synthesis (the crystallization of the hemifumarate salt) or with a high diastereomeric ratio of 96:4 but at the expense of a low yield (33%).

The invention now provides a novel route for converting the ketone containing compound according to formula (4) or its open form according to formula (11), or a mixture thereof, to the desired 5(S)-amino compound according to formula (2) or its open form according to formula (13). It is an advantage of the new process that the products are obtained in a small number of scalable steps, in a high yield and with the desired 5(S)-amino or 5(S)-amine-derivative configuration.

Thus, the invention is aimed at providing an alternative process for the preparation of a compound according to formula (13) or its ring-closed form according to formula (2), or a mixture thereof, 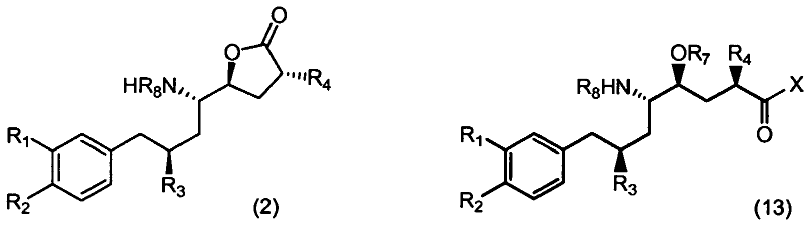
wherein R₁ being selected from the group consisting of F, Cl, Br, I, C₁₋₆halogenalkyl, C₁₋₆alkoxy C₁₋₆alkoxy-C₁₋₆alkyloxy, and C₁₋₆alkoxy-C₁₋₆alkyl;
R₂ being selected from the group consisting of F, Cl, Br,I, C₁₋₄alkyl or C₁₋₄alkoxy;
R₁ and R₂ may be linked together to form a ring structure
R₃ and R₄ each independently being branched C₃₋₆alkyl;
X stands for NHR₅ or OR₆ wherein
R₅ is C₁₋₁₂cycloalkyl, C₁₋₁₂alkyl, C₁₋₁₂hydroxyalkyl, C₁₋₆alkoxy-C₁₋₆alkyl, C₁₋₆alkanoyloxy-C₁₋₆alkyl, C₁₋₁₂aminoalkyl, C₁₋₆alkylamino-C₁₋₆alkyl, C₁₋₆dialkylamino-C₁₋₆alkyl, C₁₋₆alkanoylamino-C₁₋₆alkyl, HO-(O)C-C₁₋₁₂alkyl, C₁₋₆alkyl-O-(O)C-C₁₋₆alkyl, H₂N-C(O)-C₁₋₁₂alkyl, C₁₋₆alkyl-HN-C(O)-C₁₋₆alkyl, (C₁₋₆alkyl)₂-N-C(O)-C₁₋₆alkyl; saturated, unsaturated, or partially saturated C₁₋₁₂heterocyclyl bonded via a carbon atom to the N-atom, and which heterocyclyl is optionally substituted one or more times by C₁₋₆alkyl, trifluoromethyl, nitro, amino, *N*-mono- or *N*,*N*-di-C₁₋₆alkylated amino, C₁₋₆alkanoyl, C₂₋₆alkenyl, C₂₋₆alkynyl, C₁₋₆alkoxy, C₁₋₆alkoxycarbonylamino, C₀₋₆alkylcarbonylamino, C₁₋₆alkylcarbonyloxy, C₁₋₁₂aryl, *N*-mono or *N*,*N*-di-C₁₋₆alkylated carbamoyl, optionally esterified carboxyl, cyano, halogen, halo-C₁₋₆alkoxy, halo-C₁₋₆alkyl, C₁₋₁₂heteroaryl, saturated, unsaturated or partially saturated C₁₋₆heterocyclyl, hydroxyl, nitro; and R₆ represents H, or optionally substituted C₁₋₁₂alkyl, optionally substituted C₁₋₁₂alkylaryl, or optionally substituted C₁₋₁₂aryl;
R₇ represents H, or is an O-protecting group;

Any two of R₇, R₈, or X are optionally linked together to form a ring structure. In particular R₇ and X may be linked together to form an optionally substituted C₁₋₁₂ heterocyclic compound.

R₈ denotes H; optionally substituted C₁₋₁₂alkyl, optionally substituted C₁₋₁₂alkylaryl, or optionally substituted C₁₋₁₂aryl; optionally substituted C(O)C₁₋₆alkyl; optionally substituted C(O)OC₁₋₆alkyl; optionally substituted C(O)NHC₁₋₆alkyl; or optionally substituted C(O)N(C₁₋₆alkyl)₂; or R₈ denotes
-NHR₉;
-S(O)₂R₉; SOR₉; S(O)₃R₉; S(O)₂N(R₉);
-P(O)(R₉)₂;
-OR₁₀, with R₁₀ standing for H, optionally substituted C₁₋₆alkyl, C(O)C₁₋₆alkyl, S(O)₂R₉ or R₈ stands for (R₉)₂Y with Y being an anion such as acetate, or halogen;
and wherein each R₉ group individually represents H, optionally substituted C₁₋₁₂alkyl, optionally substituted C₁₋₁₂alkylaryl, or optionally substituted C₁₋₂aryl comprising the following steps,
a) reacting a compound according to formula (11), or its ring-closed form according to formula (4), or mixture thereof, 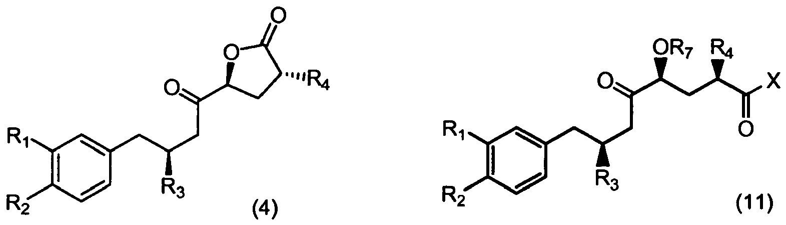 wherein R_{1,} R₂, R₃, R₄, R₇, and X are as described above for formula (2) and (13), with a compound according to formula (5)

   R₈-NH₂ (5)

   wherein R₈ is as described above for formula (2) and (13)
   which reaction results in a compound according to formula (7) or a compound according to formula (12) or a mixture thereof, 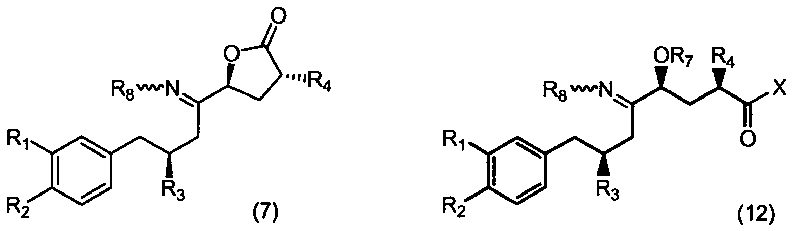 wherein R₁, R₂, R₃, R₄, R₇, R₈ and X are as described above for formula (2) and (13),
b) further reacting compound according to formula (7) or (12) or a mixture thereof, in the presence of a reducing reagent, and optionally in the presence of a catalyst, and optionally in the presence of 1 or more additives,
   which reaction results in the formation of compound according to formula (2) or formula (13) or a mixture thereof.

So the invention provides a process in which the 5(S)-amino functionality is introduced from the corresponding ketone containing compounds in fewer steps than in the processes known so far.

Preferably, in the process for the preparation of the octanoyl amides according to formula (13) with X stands for NHR₅, such as the compound Aliskiren, R₁ is 3-methoxypropoxy; R₂ is methoxy, and R₃ and R₄ are 2-propyl.

In the framework of this invention, an O-protecting group is a group as decribed in J. F. W. McOmie, "Protective Groups in Organic Chemistry", Plenum Press, London and New York 1973; or in T. W. Greene and P. G. M. Wuts, "Protective Groups in Organic Synthesis", Third edition, Wiley, New York 1999; and defined as a customary group for protecting the oxygen atom, for instance a tosylate, mesylate, benzoylate, benzoate, trialkylsilyl or carboxylic acid group, such as the acetate group, etc., and all other protecting groups customary for alcohols or oxygen atoms.

In the framework of this invention optionally substituted means that any hydrogen present in the description of R₁, R₂, R₃, R₄, R₇, R₈ R₉, R₁₀, R' and R" (and X can be replaced by another atom, hydrocarbon, or functional group known to a person skilled in the art, provided that the substituents are inert with respect to the processes carried out. For example, one, up to all hydrogens in an optionally substituted C₁₋₁₂alkyl, can be replaced by e.g. halogen, or other functional groups.

In the framework of this invention, an inert substituent is defined as a substituent that does not react itself when the desired reaction according to the invention is carried out, and that does not prevent in any other way the desired reaction from being carried out.

For example, it is known to a person skilled in the art that some substituents are very large and may sterically hinder the desired reaction from taking place, although the substituents themselves will not react.

It is understood that wherever aryl is mentioned this aryl group can also be a heteroaryl.

For all structural formulas shown in the framework of the invention, the most desired configuration is that configuration ultimately allowing the synthesis of compound according to formula (13) wherein X is NHR₅, having a 2(*S*), 4(*S*), 5(*S*), 7(*S*) configuration. However, unless specificly stated otherwise in the text, the invention also relates to racemic mixtures or scalemic mixtures of the desired compounds, whereby the desired isomer is present in excess to the undesired isomer. Preferably, the ratio of the desired isomer to the undesired isomer for any individual stereogenic centre is at least 70:30, more preferably at least 90:10, and still more preferably at least 95:5.

In an aspect of this invention the diastereomeric ratio of the compounds according to formula (2) or (13) can be improved by classical purification methods known to the person skilled in the art, such as preferential crystallization, optionally with an auxiliary, distillation, or chromatographic techniques such as simulating moving beds (SMB).

The formation of compounds according to formula (7) or (12) or a mixture thereof, from the corresponding ketone compounds formula (11), or its ring-closed form according to formula (4), or mixture thereof, using the compound according to formula (5) are performed using methods known to the person skilled in the art to prepare C=N bonds from a ketone. Suitable references can be found in March, Jerry, Chapter 6, (1985) Advanced Organic Chemistry reactions, mechanisms and structure (3rd ed.), New York: John Wiley & Sons; or other general Organic Chemistry textbooks and Comprehensive Reviews. More specific examples are given in Organic Syntheses, Coll. Vol. 9, p.610 (1998); Vol. 70, p.35 (1992) and Organic Syntheses, Coll. Vol. 6, p.818 (1988); Vol. 54, p.93 (1974). In particular these preparations of compounds according to formula (7) or (12), or a mixture thereof, are catalyzed by the addition of a Bronsted or Lewis acid, and conditions are employed in which the reaction for the preparation of the C=N bond is driven to completion by removal of water. More particular these preparations are performed by azeotropic distillation, and/or the addition of molecular sieves and/or the addition of titanium tetrachloride.

The compounds according to formula (7) or (12) or a mixture thereof, can be in the *Z* or *E* configuration, or a mixture thereof.

Preferably R₈ is a group which can be easily removed subsequently to obtain compounds according to formula (2) or formula (13) or a mixture thereof, with R₈ is equal to H. Examples of such compounds according to formula (5) with R₈ which are easily removed are optionally substituted benzylamines, electron rich anilines such as 4-methoxy aniline, optionally substituted sulfonamides, hydrazones, and phosphinylimines. Preferably, such compounds according to formula (5) with R₈ which are easily removed are optionally substituted benzylamines, more preferably α-methyl benzylamines.

Alternatively, the R₈ group is not removed, which leads to compounds according to formula (2) or formula (13) or a mixture thereof, in which R₈ is equal to R₈ in the compound according to formula (5).

The reaction of the compound according to formula (7) or (12) or a mixture thereof resulting in the formation of compound according to formula (2) or formula (13) or a mixture thereof, in the presence of a reducing reagent, and optionally in the presence of a catalyst, and optionally in the presence of an additive, can be conducted stereoselectively or non-stereoselectively. When both isomers will be formed in the same amount one speaks of a non-stereoselective reaction. Preferably the isomer of compounds according to formula (2) or formula (13) or a mixture thereof, with a (*S*)-configuration at the C-5 stereogenic center is formed in excess. For clarity reasons the obtained compounds are depicted in one configuration (C-5 stereogenic center), but it will be appreciated that the other isomer of the C-5 stereogenic center can be formed as well.

When the reaction of the compound according to formula (7) or (12) or a mixture thereof resulting in the formation of compound according to formula (2) or formula (13) or a mixture thereof, in the presence of a reducing reagent, and optionally in the presence of a catalyst, and optionally in the presence of 1 or more additives is performed non-stereoselectively, the formed isomers can be separated by methods known to the person skilled in the art, such as crystallization; classical resolution; using chromatographic techniques like simulating moving beds, optionally with a optically pure stationary phase; or by an enzymatic resolution for example with a lipase or amidase.

Preferably a stereoselective synthesis, i.e. with an excess of the (S)-configuration at the C-5 stereogenic center, preferably at least a 70:30 selectivity, more preferably at least 90:10 selectivity, most preferably a 95:5 selectivity or higher, of the compound according to formula (2) or formula (13) or a mixture thereof, in the presence of a reducing reagent, and optionally in the presence of a catalyst, and optionally in the presence of one or more additives, is performed.

Stereoselective reactions may be obtained:
A.) due to using compounds according to formula (7) or (12) or a mixture thereof with a high optical purity at the C-2, the C-4 and the C-7 stereogenic center, the formation of compound according to formula (2) or formula (13) or a mixture thereof, is stereoselective,
B.) as A but additionally by using a compound according to formula (5) which is optically enriched, hence introducing more directing chirality in the compound according to formula (7) or formula (12) or a mixture thereof. Suitable compounds are any optically enriched compound according to formula (5), preferably optically enriched amines and phenyl glycine amide are used, more preferably optically enriched α-methyl benzyl amine is used,
C.) as described under A or B but additionally in the presence of an optically enriched reducing agent,
D.) as described under A, B or C but additionally in the presence of an optically enriched catalyst.

It will be understood by the person skilled in the art that any combination of the above methods to perform a stereoselective reaction is possible.

The reducing agent can be any reducing agent, known to the person skilled in the art.

A reducing agent, also called a reductant or reducer is the element or a compound in a redox reaction that reduces another species. Here reductions refer to the addition of hydrogen (H₂),or the transfer of a hydride to a molecule.

In particular the reducing agent may be molecular hydrogen in the presence of a transition metal catalyst; alkali-metal hydrides, such as NaBH₄, NaCNBH₃, BH₃ -THF, B₂H₆ ,9-borabicyclo[3.3.1]nonane (9-BBN) or lithium tri-sec-butylborohydride (L-selectride); or hydrogen donating compounds, such as alcohols and amines, for example iso-propanol and isopropylamine, or carboxylic acids in the presence of triethylamine, for example formic acid, and salts thereof, or a Hantzsch ester; or NADPH (Nicotinamide adenine dinucleotide phosphate) and NADH (Nicotinamide adenine dinucleotide); or hydrogen donating compounds in the presence of a catalyst.

Preferably molecular hydrogen in the presence of a transition metal catalyst is used, or a hydrogen donating compound, optionally in the presence of a catalyst is used.

The reduction reaction may be carried out in the presence of one or more additives. Suitable additives are any compound which will facilitate the reduction reaction with respect to yield and/or stereoselectivity. Suitable additives are for example Lewis acids, Lewis bases, Bronsted acids, Bronsted bases, or salts, such as quaternary ammonium salts, e.g. tetrabutylammonium iodide.

The reducing agents can itself also be optically enriched, as such facilitating stereoselective reaction. Optically enriched reducing agents may be obtained by combining reducing agents like alkali-metal hydrides, such as boron hydrides, e.g BH₃, with an optically enriched compound which can coordinate to the reducing agent, such as optionally substituted amino alcohols, and optionally substituted diamines, e.g. t-BuMe₂SiOCH(Me)CH(NH₂)CPh₂OH, PhS(O)₂NHCH(Ph)CH(Ph)OH, or by a preformed optically enriched alkali-metal hydride, such as a chiral boron hydride compound as for example described by E. J. Corey, S. Shibata, R. K. Bakshi, in J. Org, Chem., 1988, 53, 2861-2863. The amount of optically enriched compound to be added to the reducing agent can be in excess, stoichiometric or catalytical amounts. Preferably stoichiometric or catalytic amounts are used, more preferably catalytical amounts are used.

In the case of molecular hydrogen as reducing agent a transition metal based catalyst is added. These transition metal based catalyst can be any source of transition metal, whether homogeneous, or heterogeneous of origin, and can be supported and non-supported, and can be non-chiral or optically enriched. Suitable catalysts are any transition metal based catalyst, preferably group VIII based catalyst, more preferably Ni, Pd, Ru, Rh, Ir and Pt based catalysts. Any support that is known in the field can be used. Suitable catalysts and conditions are for example described in Larock, R.C. Comprehensive Organic Transformations 2nd ed., Wiley-VCH, NY, 1999, pp 835-840; and by Spindler, F. and Blaser, H.U. in Handbook of Homogeneous Hydrogenation, de Vries and Elsevier (Eds.); Wiley-VCH, Weinheim, p. 1193-1214;and by Clarke, M.L. and Roff, G.J. in Handbook of Homogeneous Hydrogenation, de Vries and Elsevier (Eds.); Wiley-VCH, Weinheim, p. 437-439, and in Organic Syntheses, Coll. Vol. 3, p.827 (1955); Vol. 21, p.108 (1941).

Suitable catalyst are for example RhCl(PPh₃)₃, Pd/C, PtO₂, Raney Nickel, Ru/C, RuCl₂(PPh₃)₂(ampy) wherein ampy stands for aminomethylpyridine or Crabtree's catalyst: [Ir(1,5-cyclooctadiene)(tris-cyclohexylphosphine)(pyridine)]⁺ PF6⁻.

In the case of the reducing agent being a hydrogen donating compound the presence of a catalyst is preferred. Such catalyst can be any metal, for example sodium or magnesium, or Al(OEt)₃; a transition metal based catalyst, such as H₂IrCl₆; an organic compound (so-called organocatalyst), such as a Bronsted acid; or a biocatalyst with oxidoreductase activity (EC class 1).

Suitable catalysts and conditions are for example described by Klomp, D., Hanefeld, U. and Peters, J. in Handbook of Homogeneous Hydrogenation, de Vries and Elsevier (Eds.); Wiley-VCH, Weinheim, p. 585-632.

In the case of the use of an optically enriched catalyst in the reduction of the compound according to formula (7) or (12) or a mixture thereof resulting in the formation of compound according to formula (2) or formula (13) or a mixture thereof, the optically enriched catalyst is a transition metal based catalyst, an organocatalyst, or a biocatalyst.

Suitable transition metal based catalysts are homogeneous or heterogeneous of origin, and can be supported and non-supported. Examples of these catalysts and conditions are for example described by Spindler, F. and Blaser, H.U. in Handbook of Homogeneous Hydrogenation, de Vries and Elsevier (Eds.); Wiley-VCH, Weinheim, p. 1193-1214 in the case of molecular hydrogen as reducing agent.

In the case of hydrogen donating compounds as reducing agents, suitable optically enriched transition metal based catalysts, as well as conditions, are for example described by Blacker, A.J. in Handbook of Homogeneous Hydrogenation, J.G. de Vries and C. Elsevier (Eds.) Wiley-VCH, Weinheim, p. 1215; by Noyori et al, in Org. Biomol. Chem. 2006, 4, 393-406. Optically enriched metallocycles, such as iridacycles, and ruthenacycles are also suitable. Suitable optically enriched organocatalysts are bronsted acids, for example described by Rueping, M., Antonchick, A.P. and Theissmann, T. in Angew. Chem. Int. Ed. 2006, 45, 3683 -3686 and references therein, and suitable biocatalysts are those with oxidoreductase activity (EC class 1), for example amino dehydrogenases. In the case of biocatalysts hydrogen donating compounds are in particular NADPH (Nicotinamide adenine dinucleotide phosphate) and NADH (Nicotinamide adenine dinucleotide).

The catalyst is preferably used in quantities from 0.0001 to 10 mol-% based on the compound to be hydrogenated, the range 0.001 to 10 mol-% being especially preferred and the range 0.01 to 5 mol-% being preferred in particular.

When an enzyme is used as the catalyst the amount of enzyme used depends on the activity of the enzyme and it may vary between wide ranges. Preferably, the amount of enzyme is as low as possible. Preferably the amount of enzyme is less than 0.1 g per gram of compound to be hydrogenated, more preferably the amount of enzyme is less than 0.01 g per gram of compound to be hydrogenated, most preferably the amount of enzyme is less than 0.001 g per gram of compound to be hydrogenated.

The reduction may be carried out at low or elevated temperatures, in particular at a temperature in the range of -20 to150 °C. Preferably, the temperature is at least 10 °C, more preferably at least ambient temperature (for instance about 20°C). Preferably, the temperature is up to 120°C or less, more preferably 90°C or less.

The processes according to the invention may be carried out at atmospheric pressure or at elevated pressure. In particular the hydrogen pressure, if hydrogen gas is used as a hydrogen source, may be in the range of atmospheric to 200 bars of Hydrogen, more in particular in the range of atmospheric to 50 bars of Hydrogen.

The reduction reaction of step b) may be carried out in the absence or the presence of a solvent, wherein one solvent or a mixture of solvents may be used. Suitable solvents include aliphatic, cycloaliphatic and aromatic hydrocarbons (pentane, hexane, petroleum ether, cyclohexane, methylcyclohexane, benzene, toluene, xylene), aliphatic halogenated hydrocarbons(dichloromethane, chloroform, di-and tetrachloroethane), nitriles (acetonitrile, propionitrile, benzonitrile), ethers (diethyl ether, dibutyl ether, t-butyl methyl ether, ethylene glycol dimethyl ether, ethylene glycol diethyl ether, diethylene glycol dimethyl ether, tetrahydrofuran, dioxan, diethylene glycol monomethyl or monoethyl ether), ketones (acetone, methyl isobutyl ketone), carbonic esters and lactones (ethyl or methyl acetate, valerolactone), N-substituted lactams(N-methylpyrrolidone), carboxamides (dimethylamide, dimethylformamide), acyclic ureas (dimethylimidazoline), and sulfoxides and sulfones (dimethyl sulfoxide, dimethyl sulfone, tetramethylene sulfoxide, tetramethylene sulfone) and alcohols (methanol, ethanol, trifluoro ethanol, propanol, butanol, ethylene glycol monomethyl ether, ethylene glycol monoethyl ether, diethylene glycol monomethyl ether) and water.

In the case of a hydrogen donating compound, preferably the solvent is also used as hydrogen donating compound.

The transition metal based catalyst, optionally required for the reducing reaction with molecular hydrogen or a hydrogen donating compound, can be prepared beforehand, and added as such (vide supra), or the transition metal based catalyst can be prepared in situ, i.e. by addition of the transition metal precursor and the suitable ligands to the reaction vessel.

Suitable transition metal precursors are any available transition metal salt or complex. Preferably group VIII transition metal precursors are used, such as the one derived from Ru, Ir, Rh. Suitable precursor examples are bis(1,5-cyclooctadiene)iridium tetrafluoroborate, bis[chloro-1,5-cyclooctadiene-iridium], bis(1,5-cyclooctadiene)-rhodium tetrafluoroborate, bis(2-methylallyl)(1,5-cyclooctadiene)ruthenium, dichlorobis[(p-cymene)chlororuthenium, dichloro(1,5-cyclooctadiene)ruthenium.

Suitable ligands are any compound with possibility to donate electrons to the metal center, as known to the person skilled in the art. The ligands can be monodentate, bidentate, tridentate or tetradentate. More than one ligand can be used. The ligands can be a mixture of non chiral and chiral ligands. The amount of ligand with respect to the metal is not crucial. Preferably the amount of ligand is between 0.1 and 10, more preferably between 0.5 and 5 mol equivalents.

Suitable ligands are for example ligands described in Handbook of Homogeneous Hydrogenation, de Vries and Elsevier (Eds.); Wiley-VCH, Weinheim, 2007 or Comprehensive Asymmetric Catalysis I to III, Jacobsen, E., Pfaltz, A. and Yamamoto, H. (Eds.), Springer Verlag,1999

The invention also relates to a process for the preparation of a compound according to formula (13) or its ring-closed form according to formula (2), or a mixture thereof by reacting a compound according to formula (11), or its ring-closed form according to formula (4), or mixture thereof, with a compound according to formula (5), in the presence of a reducing reagent, and optionally in the presence of a catalyst, and optionally in the presence of an additive, without the isolation of the compounds (7) or (12). This process resulting in direct formation of the amine moiety from the ketone moiety is also often referred to as reductive amination. The same compounds according to formula (5), conditions and catalysts are used as described for the separate reaction steps as above. In general one can say that the direct reductive amination is performed with reducing agents and/or under reaction conditions that are more reactive toward imines than ketones, such as sodium cyanoborohydride (NaBH₃CN) or sodium triacetoxyborohydride (NaBH(OCOCH₃)₃). In the case of molecular hydrogen or hydrogen donating compounds as reducing agent, preferably a catalyst is added. Suitable catalysts and conditions are the same as described for the indirect reductive amination. Preferably transition metal based catalysts and biocatalysts are used. In the case of biocatalysts, enzymes like aminotransferases or aminodehydrogenases can be used. These aminotransferase enzymes can be obtained from various microorganisms for example, but not limited to, *Vibrio sp., Arthrobacter sp., Bacillus sp., Pseudomonas sp., Paracoccus sp., Rhodobacter sp..* Genes for these enzymes can be transferred and over expressed in host microorganisms like *E-Coli.* Conditions and examples of suitable aminodehydrogenases are described by Itoh et al. in J. Mol. Catal. B Enzymatic, 2000, 10, 281, in particular in combination with ammonia as compound according to formula (5).

Where an enzyme is used in the processes according to the invention, the enzyme is preferably used in combination with a suitable cofactor regeneration system for the enzyme which is known to those skilled in the art. Examples are the use of formate dehydrogenase combined with formate, or the use of glucose dehydrogenase combined with glucose. Catalytic amounts of cofactor generally suffice in these cofactor recycling systems.

In the case that amines are used as reducing agents (hydrogen donating compound), preferably the amine is the compound of formula (5), the solvent, and the reducing agent. Suitable amines for this triple use are for example phenylethylamine and 2-propylamine. When the amine is used triple (as compound according to formula (5), solvent, and reducing agent), preferably, phenylethylamine or 2-propylamine are used as compound according to formula (5), preferably an enzyme is used as catalyst, and preferably the R₈ group in the obtained compounds according to formula (2) and (13) stands for H.

The invention also relates to a process for the preparation of a compound according to formula (13) or its ring-closed form according to formula (2), or a mixture thereof by reacting a compound according to formula (11), or its ring-closed form according to formula (4), or mixture thereof, with an amine of a general formula R₈-NH₂, in the presence of a reducing agent and catalyst, preferably an enzyme, and optionally in the presence of one or more additives.

The invention also relates to a process for the preparation of 2(*S*),4(*S*),5(*S*),7(*S*)-2,7-dialkyl-4-hydroxy-5-amino-8-aryl-octanoyl amide s, (compounds according to formula (13) wherein X stands for NHR₅), or pharmaceutically acceptable salts thereof, such as the compound aliskiren, comprising reacting the compound of formula (1), 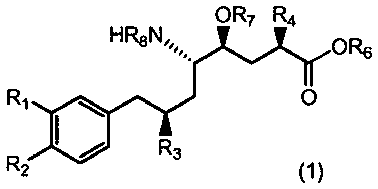 or (2) obtained by the process according to the invention with an appropriate amine, i.e. of a general formula R₅-NH₂, under conditions sufficient to form an amide bond, optionally followed by purification in order to obtain the desired configuration of the C-5 stereogenic center. Suitable conditions for the amide bond formation are known to the person skilled in the art, and are for example described in Sandham et al (Tetrahedron Letters, 2000, 41, 10091-10094).

It will be appreciated that the order of reactions to obtain certain 2(*S*),4(*S*),5(*S*),7(*S*)-2,7-dialkyl-4-hydroxy-5-amino-8-aryl-octanoyl amides, or pharmaceutically acceptable salts thereof, such as the compound aliskiren, is not crucial, so the compound according to formula (6) 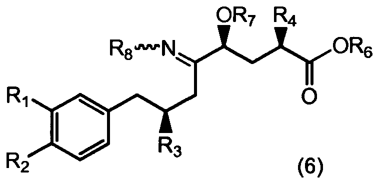 or the compound according to formula (7) can be reacted with the appropriate amine i.e. of a general formula H₂N-R₅, under conditions sufficient to form an amide bond, optionally followed by protecting the alcohol moiety at the C-4 center, resulting in the formation of compound according to formula (10), 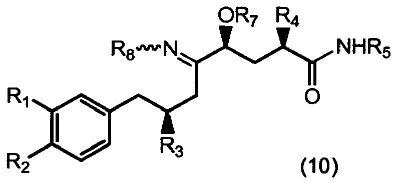 prior to the reduction reaction of the invention.

It will also be appreciated that reacting the compounds according to formula (3) 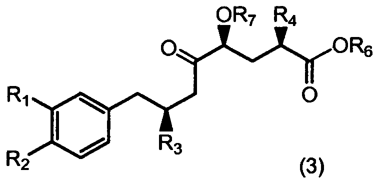 or the compound according to formula (4), or a mixture thereof, with at least two equivalents of the amine of general formula R₈-NH₂ could result in the formation of compounds according to formula (10), in which in this case R₅ is equal to R₈. In this case, the amine of general formula R₈-NH₂ is preferably the amine needed to obtain certain 2(*S*),*4*(*S*),*5*(*S*),7(*S*)-2,7-dialkyl-4-hydroxy-5-amino-8-aryl-octanoyl amides, or pharmaceutically acceptable salts thereof, such as the compound aliskiren.

It will also be appreciated that the compound according to formula (4) can be reacted with alcohols, of a general formula of HO-R₆ under conditions known to the person skilled in the art, optionally followed by protection of the alcohol moiety at the C-4 center, resulting in the formation of compounds of formula (3), prior to the reaction or reactions of the invention.

It will also be appreciated that the compound according to formula (7) can be reacted with alcohols, of a general formula of HO-R₆ under conditions known to the person skilled in the art, optionally followed by protection of the alcohol moiety at the C-4 center, resulting in the formation of compounds of formula (6), prior to the reduction reaction of the invention.

The invention also relates to new processes for the preparation of compounds according to formula (4) and (11) as described below.

The compound according to formula (4), with R₁ is methoxypropoxy, R₂ is methoxy, and R₃ and R₄ are 2-propyl can be obtained by a method known in the literature, as described in Rueger et al referred to above.

This lengthy route has several drawbacks, of which the use of stoichiometric amounts of chiral auxiliaries, a low diastereoselectivity, and hence a cumbersome separation, are the most striking ones.

An other aspect of the invention is a new process to compounds according to formula (3) or formula (4) or mixture thereof, which are relatively short, high yielding and with high selectivities.

The surprisingly short and efficient synthesis for the compounds according to formula (3) and (4), comprises the steps of
a) an addition reaction of a cyanide to the enantiomerically enriched compound according to formula (14), wherein R₄ is as described in claim 1, followed by
b) a hydrolysis of the nitrile group followed by acid chloride synthesis
c) a coupling reaction of the acid chloride compounds with compound according to formula (19).

For clarity reasons the syntheses are depicted in scheme I:

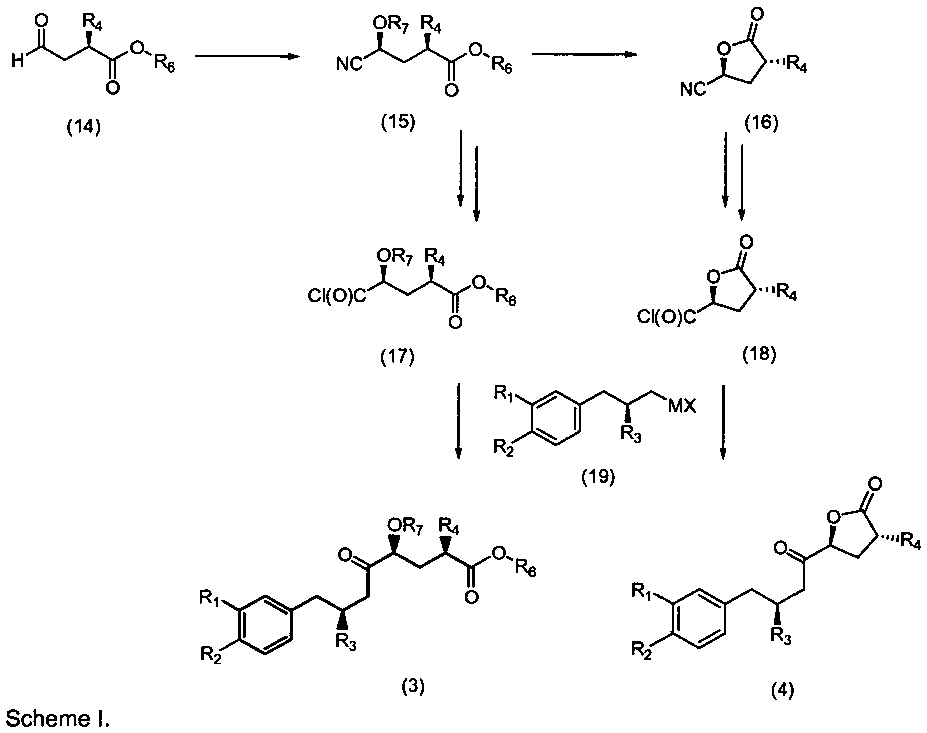

The coupling reaction of the compounds according to formula (18) and (19), with R₁ is methoxypropoxy, R₂ is methoxy, R₃ and R₄ are 2-propyl and MX is MgCl, leading to compound according to formula (4) has been described in Rueger et al, as referred to earlier. Similar conditions could be used to achieve the coupling between compounds according to formula (17) and (19), leading to compound according to formula (3), wherein M is chosen from the group of Mg, Li, Ce, Ti, Cu, Zn, Mn, Fe, B, Si, or Al, and X is an anion, in general a halide. Preferably M is Mg, and X is chloride.

Compounds according to formula (17) and (18) or mixture thereof can be easily obtained from the corresponding nitrile containing compounds according to formula (15) and (16) or mixture thereof, using methods known to the person skilled in the art (hydrolysis of nitriles and acid chloride formation, respectively).

Compounds according to formula (15) and (16) (as disclosed in WO2009/080773) or mixture thereof are prepared by reacting the chiral aldehyde of formula (14) with a cyanide, preferably with HCN, NaCN, KCN, (R)₃SiCN (with R selected from C₁₋₆alkyl, C₁₋₁₀alkylaryl, and C₁₋₁₀aryl), optionally in the presence of a chiral catalyst. Said catalyst can be a chiral organic compound, a chiral metal complex, or an enzyme, as described in by F.X. Chen and X.M. Feng in "Asymmetric synthesis of cyanohydrins" Current Organic Synthesis 2006, 3, 77-97, and references therein; and by P. Poechlauer, W. Skranc, and M. Wubbolts in "The large-scale biocatalytic synthesis of enantiopure cyanohydrins" in Asymmetric Catalysis on Industrial Scale; H.U. Blaser and E. Schmidt, Eds. Wiley-VCH, 2004, pp 151-164. Preferably HCN, or (R)₃SiCN in the presence of a suitable chiral catalyst is used. More preferred HCN and the enzyme HNL (hydroxynitrile lyase) are used. Suitable conditions for the synthesis of compound of formula (15) or compound according to formula (16), or a mixture thereof, are known to the person skilled in the art and are described in the references above, and references therein.

The compound according to formula (15) with R₇ is equal to H is ring-closed relatively easily to the compound according to formula (16) by employing acidic conditions e.g. catalytic amount of para-toluene sulfonic acid, all in analogy with lactonization methods known to the person skilled in the art. For the lactone formation, R₆ is preferentially C₁₋₆alkyl, more preferentially R₆ is methyl.

The compound according to formula (16) in the diastereochemically desired configuration can be obtained by ring-closing the compound according to formula (15) in the desired configuration, or by ring-closing both diastereomers of the compound according to formula (15) with fixed configuration at C-2 stereogenic center, followed by epimerization of the C-4 stereogenic center to the thermodynamically preferred diastereomer, also being the desired diastereomer. Said epimerisation can be conducted by heating the compound according to formula (16), optionally in a suitable solvent, and optionally in the presence of a base or other suitable additives. Alternatively, the diastereoisomer with the desired configuration can be separated from the other diastereoisomer making use of their different physical properties (e.g. preferential crystallization), or by means of classic or separating moving beds (SMB) chromatography. Suitable examples of SMB chromatography can be found in Schulte and Strube J. Chromatogr. A 2001, 906, 399-416 and references therein.

Preferably the compound according to formula (16) in the diastereochemically desired configuration is obtained by ring-closing an optically pure compound according to formula (15) with R₇ is equal to H and R₆ is C₁₋₆alkyl.

Alternatively, diastereomeric mixtures of compounds according to formula (15) or (16) can be hydrolysed to the corresponding acids and than purified with respect to the undesired diastereomer by using purification methods known to the person skilled in the art, e.g. preferential crystallization, optionally in the presence of a chiral auxiliary, or chromatographic techniques such as SMB.

In another aspect, the invention provides new routes to the useful building block according to formula (14), which was disclosed in WO2009/080773. The compound according to formula (14) can be obtained in various alternative ways, all surprisingly short, and atom-efficient, as illustrated in Scheme II for the compound according to formula (14) with R₄ is equal to 2-propyl (numbered as 14a), wherein R₆ is as described for compounds according to formula (13), R' and R" are equal or independently stand for an optionally substituted C₁₋₈alkyl, or form together an optionally substituted ring of maximal 10 carbon atoms, and Hal stands for Halogen.

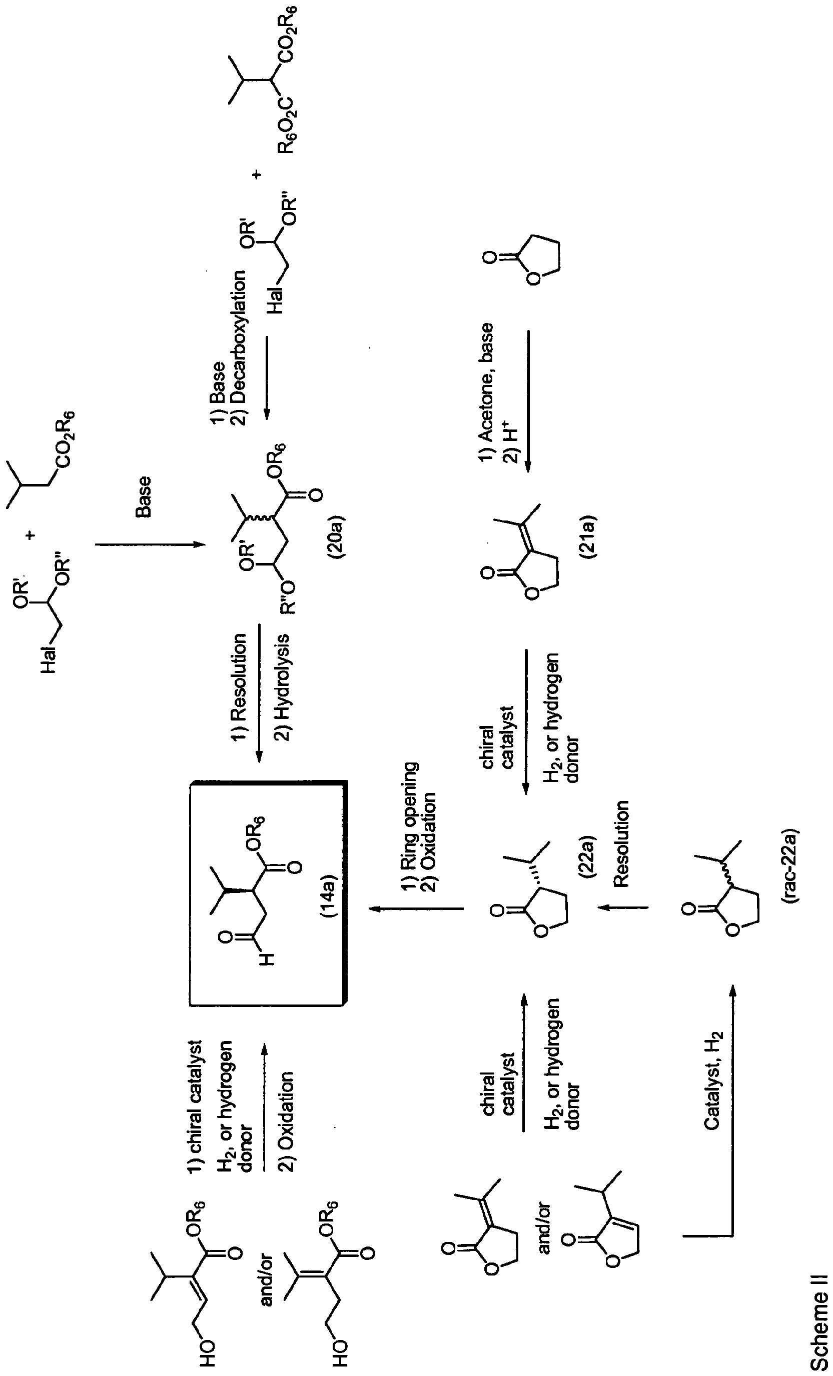

As part of the invention new routes to compounds according to formula (14) are disclosed.

In one of the new routes, the compound according to formula (14) is obtained by resolution methods of the corresponding acetal, the compound according to formula (20) (depicted in the scheme for compound of formula (20a)), followed by hydrolysis of the acetal moiety to the aldehyde moiety, for example with acid such as aqueous HCl. Suitable resolution methods are those known to the person skilled in the art, such as preferential crystallization, optionally with the aid of chiral auxiliaries; classical resolution; chromatographic techniques such as SMB; or enzymatic resolution methods. In particular, enzymatic resolution methods are used. Suitable enzymes to be used in resolution processes as described above are for example hydrolases. Examples of suitable hydrolases are esterases, lipases, proteases, peptidases or acylases. These enzymes may be obtained from animals, for example pig liver esterase, or from microorganisms, such as bacteria or fungi or from plants.

Examples of suitable enzymes are disclosed in e.g. WO2006/117057, in particular on p.4 line 25 to p.7, line 6. Preferably, enzymes are used of non-animal origin.

The racemic compound according to formula (20) can be obtained in several ways, for example by alkylating the substituted malonate ester with an alpha-halogenated acetal, followed by hydrolysis and decarboxylation as depicted on the right side of Scheme II. Conditions and reagents for these steps are known to the person skilled in the art. Alpha-alkylating an appropriate ester with the alpha-halogenated acetal, as depicted on the top of Scheme II is another viable option.

Other new routes to compound according to formula (14) are based on catalytic asymmetric reductions of cyclic or non cyclic precursors in which the C=C double bond can be a tetrasubstituted one, or the isomerized tri-substituted one, or a mixture thereof, optionally followed by ring opening of the lactone, and subsequent oxidation.

For the cyclic compounds according to formula (21a) this reaction sequence is depicted on the center-bottom of Scheme II. For the isomerized cyclic compound, or a mixture thereof with compound according to formula (21a) this is depicted on the center-left of scheme II for the compounds with R₄ is 2-propyl. For the non cyclic precursors this reaction sequence is depicted on the top-left of scheme II (for R₄ is 2-propyl).

Suitable conditions and reagents for the catalytic asymmetric reductions of the C=C bond are similar as described above for the reduction of the C=N bond. More specific examples are described in chapters 23-31 of Handbook of Homogeneous Hydrogenation, de Vries and Elsevier (Eds.); Wiley-VCH, Weinheim, 2007

Preferably molecular hydrogen in combination with optically enriched transition metal based catalysts are used for the reduction of the C=C bond.

Suitable conditions and reagents for the ring-opening of the lacton, and subsequent oxidation of the alcohol moiety are known to the person skilled in the art. Preferably the ring-opening of the lacton (the compound with R₄ is 2-propyl is depicted in the scheme with formula 22a) is performed in non alcoholic solvents.

Another new route to compound according to formula (14) is any resolution method of the racemic lacton (a compound according to formula (22)), itself easily obtained by reduction of the compound according to formula (21), or its isomerised compound, or a mixture thereof, as depicted at the left-bottom corner of Scheme II for the compounds with R₄ is 2-propyl. Suitable resolution methods for this route are similar ones as described above for compound according to formula (20).

For the synthesis of the compound according to formula (14), preferably,
a. resolution methods of the compound according to formula (20), followed by hydrolysis of the acetal moiety to the aldehyde moiety, and
b. the catalytic asymmetric reduction of compound according to formula (21), followed by ring opening and oxidation,
are used.

More specific, and without being limited thereto, the synthesis to Aliskiren (2(S),4(S),5(S),7(S)-N-(2-carbamoyl-2-methylpropyl)-5-amino-4-hydroxy-2,7-diisopropyl-8-[4-methoxy-3-(3-methoxypropoxy)fenyl]-octanamide hemifumaraat) using reactions of the invention, is as depicted in scheme III.

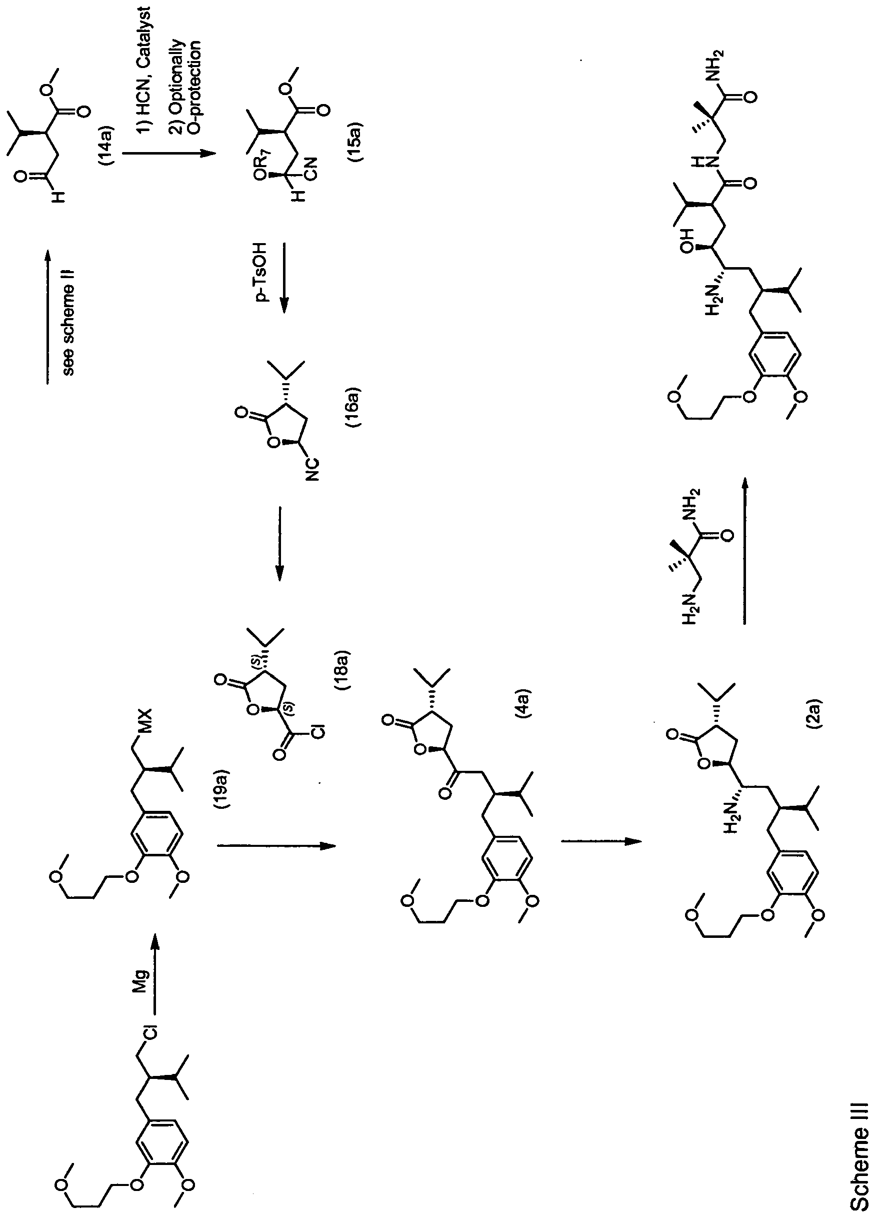

The processes and compounds according to the invention are particularly useful for the preparation of 2(*S*),4(*S*),5(*S*),7(*S*)-2,7-diaikyl-4-hydroxy-5-amino-8-aryl-octanoyl amides (compounds according to formula 13 with X stands for NHR₅), or pharmaceutically acceptable salts thereof, such as disclosed in WO02/02508, WO 2006/061427 and WO 2006/095020, which are hereby incorporated by reference. In particular, the processes and compounds according to the invention are useful in the preparation of compounds according to formula (I) of claim 1 of WO02/02508 A1 and the pharmaceutically acceptable salt thereof.

Salts, including pharmaceutically acceptable salts of compounds according to formula (13), wherein X is HNR₅, means salts that are generally safe, nontoxic and neither biologically nor otherwise undesirable, and that possess the desired pharmacological activity of the parent compound. These salts are derived from an inorganic or organic acid or base.

Pharmaceutically acceptable salts of the compounds according to formula (13), are for example disclosed in US5,559,111 which is hereby incorporated by reference and in particular in column 11 line 50 to column 12 line 33, of said document, which paragraph is also explicitly incorporated by reference.

All compounds according to the invention may be isolated by methods known to the person skilled in the art, such as crystallization, distillation, or chromatographic techniques. Some specific isolations, but not limited hereto, are described in the examples.

The amount of reagents and additives used in the processes of the invention are in principal known to the person skilled in the art and may vary between wide limits. Depending on the nature of the reagent (reactivity, costs, etc.), it can be used in large excess, or in stoichiometric amounts, or less than stoichiometric amounts, for example, it wil be understood that catalysts and certain additives will only be used in less than stoichiometirc amounts, in particular in catalytic amounts.

The following examples illustrate the invention, without however being limited hereto.

### Preparative EXAMPLE 1

### Preparation of 2-isopropylidene-γ-butyrolactone from acetone and γ-butyrolactone:

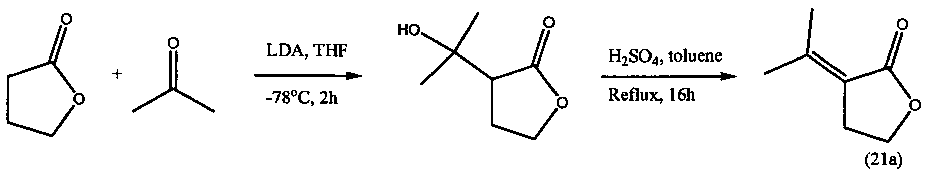

Diisopropylamine (41.0 mL, 292 mmol, 1.15 eq.) was dissolved in THF (140 mL) and cooled to -70°C. *n*-BuLi (2.9 M in hexanes, 95.8 mL, 278 mmol, 1.1 eq.) was added dropwise at -70°C and the resulting yellow solution was stirred at 0°C for 30 minutes. The solution was then cooled to -70°C, after which a solution of γ-butyrolactone (19.3 mL, 253 mmol, 1.0 eq.) in THF (100 mL) was added dropwise over 20 minutes. The solution was stirred for 1 hour at -70 °C and then acetone (37.2 mL, 506 mmol, 2.0 eq.) was added dropwise over 20 minutes. Subsequently, the reaction was stirred for 4 hours at -70 °C and then allowed to reach room temperature while stirring overnight. The reaction was quenched with water and diluted with MTBE. The organic layer was extracted with HCl (aq. 1 N, 6x) and the combined aqueous layers were extracted with chloroform (9x). The combined organic layers were dried (Na₂SO₄), filtered and concentrated *in vacuo* to give the alcohol as a yellow liquid/oil which was used in the next step without further purification.

The crude alcohol was dissolved in toluene (500 mL) and H₂SO₄ (95-97%, 1.75 mL, 32.8 mmol, 13 mol%) was added. The resulting reaction mixture was heated under reflux overnight. Subsequently, the reaction mixture was allowed to reach room temperature. The organic layer was diluted with MTBE and washed with HCl (aq. 1 N), NaHCO₃ (sat. aq.) and brine (sat. aq.), dried (Na₂SO₄), filtered and concentrated *in vacuo* to give a brown liquid. The aqueous layers were combined and the pH was adapted to 7. The aqueous layer was then extracted with dichloromethane (3x) and the combined organic layers were dried (Na₂SO₄), filtered and concentrated *in vacuo* to give a yellow liquid that was added to the previously obtained brown fraction. The product was purified by vacuum distillation (65°C, 1 mbar) to give a slightly yellow liquid. After further purification by column chromatography (heptane-EtOAc 7:3) the desired product (21 a) (22.0 g, 174 mmol, 69%) was obtained as a colorless liquid.
¹H-NMR of compound according to formula (21 a) (CDCl₃, 300 MHz) δ = 1.89 (s, 3H, CH₃), 2.26 (s, 3H, CH₃), 2.88 (m, 2H, C3-H₂), 4.29 (t, *J* = 7.5 Hz), C4-H₂) ppm.

### Preparative EXAMPLE 2

### Racemic 2-isopropyl-γ-butyrolactone from 2-isopropylidene-γ-butyrolactone:

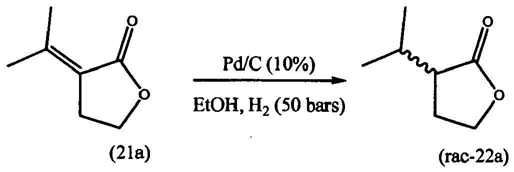

The tetrasubstituted alkene (21 a) (5.0 g, 40 mmol) was dissolved in ethanol (100 mL) and stirred in an autoclave under an hydrogen atmosphere (50 bars) in the presence of Pd/C (500 mg) for 16 hours at room temperature. Subsequently, the suspension was filtered over dicalite and the resulting clear solution was concentrated *in vacuo* to give the saturated lacton (3.1 g, 24 mmol, 61%) as a colorless liquid.
¹H-NMR of compound according to formula (rac-22a) (CDCl₃, 300 MHz) δ = 0.94 (d, *J* = 6.6 Hz, 3H, CH₃), 1.05 (d, *J* = 6.6 Hz, 3H, CH₃), 2.08 (m, 1H, C3-H), 2.21 (m, 2H, C2-H and C3-H), 2.48 (m, 1H, (CH₃)₂C*H*), 4.18 (ddd, *J* = 7.2, 9.0, 9.0 Hz, 1H, C4-H), 4.31 (ddd, *J* = 3.3, 8.7, 8.7 Hz, 1H, C3-H) ppm.

### Preparative EXAMPLE 3

### Opening of racemic 2-isopropylidene-γ-butyrolactone to the sodium salt:

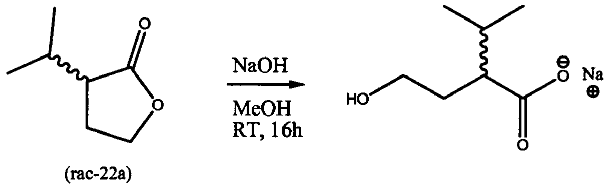

To a solution of compound (rac-22a) (1.5 g, 12 mmol) in MeOH (10 mL) was added a solution of NaOH (0.47 g, 12 mmol, 1.0 eq.) in MeOH (20 mL). The resulting solution was stirred at room temperature for 16 hours and then concentrated *in vacuo* to give the corresponding racemic sodium salt in quantitative yield as an off-white foam.
¹H-NMR of sodium salt (CD₃OD, 300 MHz) δ = 0.94 (d, *J* = 6.4 Hz, 3H, CH₃), 0.98 (d, *J* = 6.4 Hz, 3H, CH₃), 1.70-1.87 (m, 3H, C2-H and C3-H₂), 1.95 (m, 1H, (CH₃)₂C*H*), 3.58 (m, 2H, C4-H₂) ppm.

### Preparative EXAMPLE 4

### Opening of optically enriched 2-isopropylidene-γ-butyrolactone to the sodium salt, followed by ring closure to determine the e.e. (of the sodium salt)

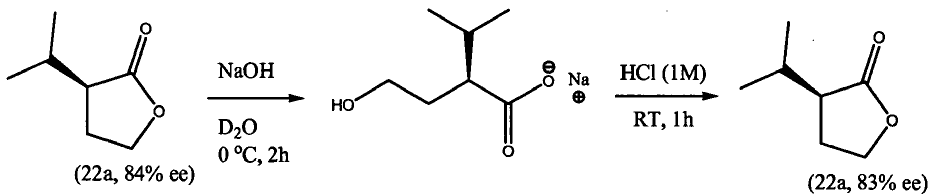

Optically enriched lactone (22a) (110 mg, 0.86 mmol, 84% ee) is dissolved in D₂O (1.0 mL) and a solution of NaOH (34.4 mg, 0.86 mmol, 1.0 eq.) in D₂O (1.0 mL) is added at 0 °C over 30 minutes in three portions. The reaction mixture is stirred for an additional 90 minutes at 0°C. ¹H-NMR shows complete conversion into the linear ring opened compound. The pH of the reaction mixture is adapted to 0-1 using aqueous HCl (1 M) and the reaction mixture is stirred for 1 hour at ambient temperature. The aqueous layer is extracted with chloroform (2x) and the combined organic layers are dried (Na₂SO₄), filtered and concentrated *in vacuo.* Chiral GC shows that the enantiomeric excess of compound according to formula (22a) is 83%.

### Preparative EXAMPLE 5

### Alkylation of the sodium salt to the methylester:

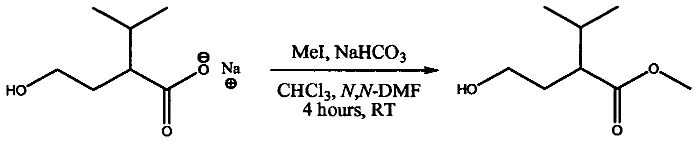

The sodium salt of 4-hydroxy-2-isopropylbutanoate (56 mg, 0.33 mmol) and NaHCO₃ (s, 277 mg, 3.3 mmol, 10 eq.) were suspended in a mixture of chloroform (2.5 mL) and DMF (1.0 mL). The resulting suspension was stirred at room temperature for 4 hours and then quenched by addition of water. The aqueous layer was extracted with chloroform (3x) and the combined organic layers were dried (Na₂SO₄), filtered and concentrated *in vacuo.* Residual DMF was removed by coevaporation with toluene to give the desired methyl ester as a colourless oil (quantitative).
¹H-NMR of methyl ester (CDCl₃, 300 MHz) δ = 0.93 (d, *J* = 5.4 Hz, 3H, CH₃), 0.95 (d, *J* = 5.4 Hz, 3H, CH₃), 1.65-1.93 (m, 4H, C2-H, C3-H₂ and OH), 2.32 (m, 1H, (CH₃)₂C*H*), 3.64 (m, 2H, C4-H₂), 3.69 (s, 3H, CH₃OCO) ppm.
¹³C-NMR of methyl ester (CDCl₃, 75 MHz) δ = 20.0, 20.4, 30.5, 32.2, 49.3, 51.4, 61.4, 176.3 ppm.

### Preparative EXAMPLE 6

### Oxidation of the 4 -hydroxy-ethylester to the corresponding aldehyde (14):

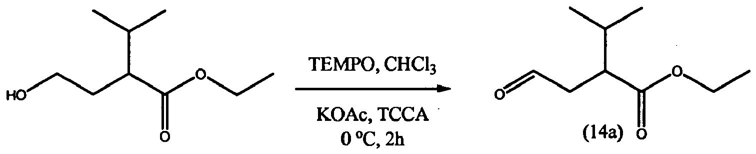

To a solution of the 4-hydroxy ethyl ester (50 mg, 0.29 mmol) in chloroform (2.5 mL) was added KOAc (42 mg, 0.43 mmol, 1.5 eq.). The resulting suspension was cooled to 0 °C and then TEMPO (1.0 mg, 6.4 µmol, 2.2 mol%) and finally trichlorocyanuric acid (TCCA, 34 mg, 0.15 mmol, 0.50 eq.) were added in a single portion. The resulting reaction mixture was stirred at 0 °C for 90 minutes and then quenched with Na₂S₂O₃ (aq. 10% w/w). The aqueous layer was extracted with dichloromethane and the combined organic layers were washed with NaHCO₃ (sat. aq.), dried (Na₂SO₄), filtered and concentrated *in vacuo* to give compound (14a) (30 mg, 0.17 mmol, 61%) as a yellow oil.
¹H-NMR of (14a) (CDCl₃, 300 MHz) δ = 0.92 (d, *J* = 6.6 Hz, 3H, CH₃), 0.94 (d, *J* = 6.6 Hz, 3H, CH₃), 1.26 (t, *J* = 7.2 Hz, 3H, C*H*₃CH₂OCO), 2.03 (m, 1H, (CH₃)₂C*H*), 2.53 (m, 1H, C4-H), 2.78 (m, 1H, C2-H), 2.88 (m, 1H, C4-H), 4.16 (m, 2H, CH₃*CH*₂OCO), 9.79 (s, 1 H) ppm.

### Preparative EXAMPLE 7

### Preparation of methyl-4,4-diethoxy-2-isopropylbutanoate (a compound according to formula 20a)

Sodium hydride (32.0 g of a 60% dispersion in mineral oil; 0.8 mole) was suspended in 1 L dry DMF and cooled to 0-5°C by external cooling. Next dimethyl 2-isopropylmalonate (139.2 g; 0.8 mole) was added in 1 hour at 5-10°C and stirred for another hour at 10°C, whereafter no further evolution of hydrogen could be detected. Next bromoacetaldehyde diethylacetal (157.6 g; 0.8 mole) was added to the reaction mixture to give a red-brown solution, which was then heated to 130°C for 18 hrs with stirring. GC analysis (HP-5, 30m * 0.32 mm * 0.25 µm; Tᵢₙᵢₜ=80°C (1 min), rate 20°C/min, T_{end}=300°C(3 min)) showed complete conversion of the malonate.

This mixture is cooled to room temperature methanol (32 g; 1 mole) and lithiumchloride (34 g; 0.8 mol) was added. The mixture was stirred under nitrogen and heated to 130°C for 8 hours. GC analyses showed complete conversion. Next, the reaction mixture is concentrated in vacuo (70°C, 25 mbar, ∼500 ml distillate), cooled and after addition of water (500 ml) and methyl-tert.butylether (300 ml), filtered over a precoated filter. The phases were separated and the water layer was extracted with methyl-tert.butylether (2 x 300 ml). The combined organic layers were dried over Na₂SO₄ and concentrated by evaporation of the solvent under reduced pressure (70°C, 25 mbar). The residual liquid was subjected to fractional distillation (60°C, 1 mbar) to give 82 g of colorless oil of the racemic compound according to formula (20a). Overall yield = 45%.
¹H-NMR (CDCl₃): 0.9 (d, 6H); 1.2 (m, 6H); 1.7 (m, 1H); 1.8-2.1 (m, 2H); 2.3 (m, 1H); 3.4-3.7 (m, 4H); 3.7 (s, 3H); 4.4 (m, 1H)

### Preparative EXAMPLE 8

### Enzymatic resolution of methyl-4,4-diethoxy-2-isopropylbutanoate (a compound according to formula 20a)

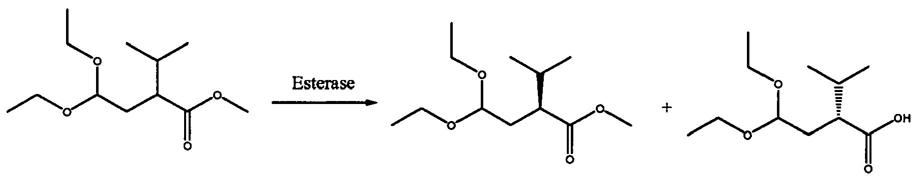

In a round-bottom flask equipped with a pH-STAT apparatus 47 g of the purified methyl 4,4-diethoxy-2-isopropylbutanoate (202.3 mmol) were added to 2.265 I of Phosphate Buffer pH 7.5 (100 mM). 85 ml of the cell-free extract of the enzyme (Diversa 13665, 5.35 g of enzyme) were then added and the solution was stirred at room temperature until one of the enantiomers was totally consumed (monitored by the consumption of NaOH used to keep the pH constant).

After 72 hours (e.e. > 99% of the remaining methyl 4,4-diethoxy-2-isopropylbutanoate), MTBE (500 ml), Charcoal (20 g) and Celite (20 g) were added and the mixture was stirred for 10 minutes. The suspension is then filtered and the water phase extracted three times with MTBE (3 X 500 ml). The combined organic phases were filtered over Celite to eliminate the residual biomass, and the organic phase was dried over anhydrous sodium sulphate. After filtration the solvent was evaporated *in vacuo* to give the desired enantiomer of methyl 4,4-diethoxy-2-isopropylbutanoate (e.e. > 99%) as a pale yellow oil (20.5 g, 88.2 mmol, 95% pure - based on GC analysis).

### Preparative EXAMPLE 9

### Preparation of methyl 2-(formylmethyl)-3-methylbutanoate (14a)

Methyl-4,4-diethoxy-2-isopropylbutanoate (e.e. > 99%, 25.6 g; 110 mmol) is dissolved in 220 ml 0.5N HCl and stirred for 2 hrs at room temperature. The mixture is extracted with methyl-t-butylether (2 x 100 ml) and concentrated by evaporation of the solvent under reduced pressure to yield 16.3 g of colorless oil.
¹H-NMR (CDCl₃): 0.95 (dd, 6H); 1.85-2.00 (m, 1H); 2.35-2.45 (dd, 1H); 2.65-2.70 (m, 1H); 2.75-2.90 (m, 1H); 3.60 (s, 3H); 9.70 (s, 1H)

### Preparative EXAMPLE 10

### Preparation of 2-isopropyl-γ-butyrolactone (compound according to formula 22a) by catalytic asymmetric hydrogenation of dihydro-3-(propan-2-ylidene)furan-2(3h)-one (21a)

5.7 mg (11 µmol) (R)-1-[(S)-2-(Di-2-furfurylphosphino)-ferrocenyl]ethyldi-tert-butylphosphine and 250 µl 0.04 M (10 µmol) Ru(COD)(methylallyl)₂ solution in dichloromethane were placed in a vial followed by 1.4 µl (10 µmol) HBF₄.OEt₂, 5 ml dichloromethane and 35 µl (320 µmol) dihydro-3-(propan-2-ylidene)furan-2(3H)-one. This solution was transferred to the hydrogenation apparatus and was hydrogenated for 1 h 30 min at 50 °C and 25 bar hydrogen. GC-analysis showed 100% conversion and 93% enantiomeric excess.

### GC CONDITIONS:

Column Chiraldex G-TA (30 m x 0.25 mm ID x 0.13 µm)
Oventemperature 80°C (1 min) → 5°C/min → 180°C (5 min)
Carrier gas He flow rate 1.2 ml/min
Split ratio 1: 50
Injection volume 1 µL
Enantiomer 1:10.3 min
Enantiomer 2: 10.8 min
Substrate: 11.7 min

### Preparative EXAMPLE 11

### Synthesis of enantiomerically enriched cyanohydrin: (S)-methyl 2-((S)-2-cyano-2-hydroxyethyl)-3-methylbutanoate (a compound according to formula 15)

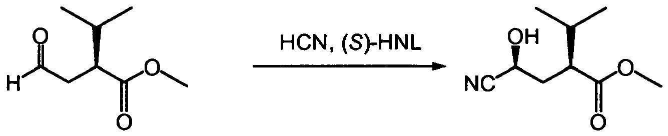

A mixture of 72 ml S-HNL in 18 ml K-Phosphate buffer (20 mM, pH=5.6) and 360 ml MTBE is charged to the reactor. The 2 phases are mixed by stirring and at a temperature of 0°C 20 ml pure HCN (0.53 mol) is added. Methyl 2-(formylmethyl)-3-methylbutanoate (16.5 g) diluted with 95 ml MTBE are dosed to the reactor in 20 minutes. The mixture is stirred for 3 hours at 0°C. The conversion of the aldehyde is >95% and e.e. >95%.

The reaction mixture is diluted with 200 ml MTBE. After phase separation the water layer is extracted with MTBE (2 x 100 ml), acidified with 0.2 ml concentrated H₃PO₄ and dried over Na₂SO₄. After filtration the MTBE is evaporated under reduced pressure yielding 21.0 g of the cyanohydrin of formula (15) (e.e. of C-4 center is 97%).

### Preparative EXAMPLE 12

### Synthesis of the lactone nitrile: (2S,4S)-tetrahydro-4-isopropyl-5-oxofuran-2-carbonitrile (a compound according to formula 16)

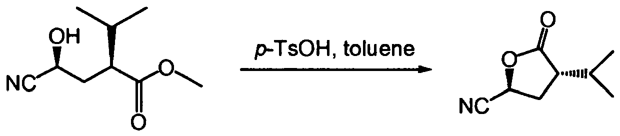

The cyanohydrin of example 11 (12.4 g) was diluted with 120 mL of toluene and 25 g of mol sieves 5A were added. To this mixture, 250 mg of p-toluenesulfonic acid was added and, whilst stirring, the mixture was heated at 70 °C for 1 hour. After cooling to RT, the molecular sieves were filtered off and washed with toluene. The collected organic phase was washed with a saturated aqueous solution of sodium bicarbonate and dried over Na₂SO₄. After filtering off the Na₂SO₄, the organic phase was concentrated under reduced pressure yielding 10.7 g of crude lactone. Purification by flash column chromatography on silica gel yielded the title compound with >98% purity.
¹H-NMR (400 MHz, CDCl₃): δ 5.11 (dd, *J*= 8.4, 2.3 Hz, 1H), 2.84-2.74 (m, 1H), 2.62-2.41 (m, 2H), 2.31-2.16 (m, 1H), 1.10 (d, *J*= 6.9 Hz, 3H), 0.97 (d, *J*= 6.9 Hz, 3H).

### Preparative EXAMPLE 13

### Synthesis of the TBS-Protected cyanohydrin: (S)-methyL 2-[(S)-2-cyano-2-(t-butyldimethylsilyl)oxyethyl]-3-methylbutanoate, compound according to formula (15)

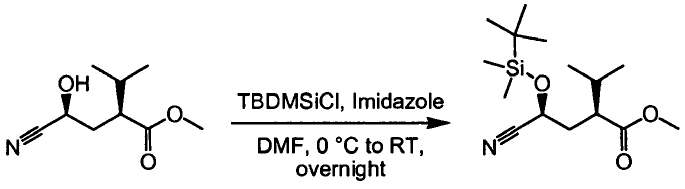

Imidazole (7.35 g, 108 mmol) was added at 0 °C to a solution of the cyanohydrin (10 g) in DMF (180 mL), followed by TBDMSiCl (9.77 g, 64.8 mmol). The mixture was stirred at room temperature overnight. Subsequently, the reaction mixture was poured over an ice-cold aqueous HCl solution (1 M, 80 mL). After the addition of diethyl ether (100 mL), the organic layer was separated and the aqueous layer was further extracted with diethyl ether (2x100 mL). The combined organic layers were washed with a saturated aqueous solution of sodium bicarbonate and brine, dried over Na₂SO₄ a the solvent was then removed under reduced pressure. The crude mixture was purified by flash column chromatography on silica gel yielding the title compound.
¹H-NMR (400 MHz, CDCl₃): δ 4.26 (dd, *J*= 9.2, 3.4 Hz, 1H), 3.50 (s, 3H), 2.37-2.29 (m, 1H), 2.08-1.96 (m, 1H), 1.86-1.68 (m, 2H), 0.81-0.68 (m, 15H), 0.00 (s, 3H), -0.08 (s, 3H).

### Preparative EXAMPLE 14

### Hydrolysis of tetrahydro-4-isopropyl-5-oxofuran-2-carbonitrile (a compound according to formula16) to the corresponding acid: tetrahydro-4-isopropyl-5-oxofuran-2-carboxylic acid

Tetrahydro-4-isopropyl-5-oxofuran-2-carbonitrile (19.7 g, 128.7 mmole) was dissolved in 200 ml 6N HCl and stirred at reflux for 18 hrs. The mixture was cooled to room temperature and extracted with methyl-tert.butylether (3 x 100 ml). The combined organic layers were dried over Na₂SO₄, filtered, and concentrated by evaporation of the solvent under reduced pressure to yield 16.8 g of yellow oil (97.7 mmol).

### Purification via the dicyclohexylamine-salt:

The yellow oil is dissolved in 240 ml heptane/ methyl-tert.butylether (1 : 2 v/v). Next dicyclohexylamine (17.7 g; 97.7 mmole) is added dropwise. Spontaneous crystallization occurs. The precipitate is isolated by filtration, washed with heptane and dried in vacuo to yield 17.6 g of a white solid. Next, this white solid is suspended in 200 ml methyl-tert.butylether and 4.9 ml (58.8 mmole) of conc HCl is added drop wise at room temperature. The mixture is stirred for another 0.5 hrs. The precipitate is filtered off and the filtrate is concentrated in vacuo to yield 9.8 g of the acid (57.0 mmol) as a light brown oil which crystallizes after a few hours standing.
¹H-NMR (CDCl₃): 0.95 (d, 3H); 1.05 (dd, 3H); 2.2-2.7 (3 x m, together 4H); 9.2 (broad s, 1H)

### Preparative EXAMPLE 15

### Synthesis of tetrahydro-4-isopropyl-5-oxofuran-2-carbonyl chloride (a compound according to formula 18)

A dry and nitrogen gas filled 3 necked round bottom flask (250 ml) equipped with magnetic stirrer, nitrogen gas inlet, septum and thermocouple, was filled with 7.12 g (38.08 mmol, 1.00 eq) carboxylic acid lactone (of example 14) and 45 ml toluene (dry, degassed). 5 ml (7.5 g, 59.09 mmol, 1.6 eq) oxalylchloride was added and subsequently 22 µl dimethylformamide (dry) resulting in gas evolution. The solution was stirred at room temperature for 3.5 h after which a sample quenched in ethanol showed no starting material by TLC. The solvent was removed in vacuum at room temperature and giving the desired acid chloride.

### Preparative EXAMPLE 16

### Preparation of compound according to formula (19a)

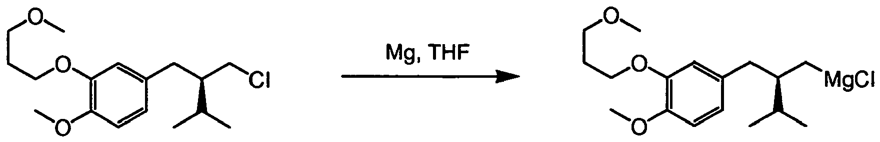

A dry, nitrogen gas filled, 3 necked round bottom flask (500 ml) equipped with reflux condenser, dropping funnel, magnetic stirrer and nitrogen gas inlet was filled with 4.680 g (192.5 mmol, 1.1 eq) Mg powder, 10 ml tetrahydrofuran (THF, dry, degassed)) and a crystal of iodine. A solution of 54.930 g (174.5 mmol, 1.0 eq) 2-(3-methoxypropoxy)-4-((R)-2-(chloromethyl)-3-methylbutyl)-1-methoxybenzene, and 0.6 ml 1,2-dibromomethane in 163 ml THF was prepared and 20 ml of this solution was added to the Mg slurry. This mixture was heated to boil, and while keeping it refluxing, the remaining solution of above was added drop wise in 1.5 h. The mixture was then heated under reflux conditions for another 3.5 h, after which analysis by TLC showed no starting material. The concentration of the Grignard solution was 0.763 M determined by titration with s-butanol using phenantroline as indicator.

### Preparative EXAMPLE 17

### Synthesis of compound according to formula (4a): Coupling of compound according to formula (19a) with the compound according to formula (18a).

The in example 15 prepared acid chloride (18a) was dissolved in 45 ml THF (dry, degassed) and cooled with an ice/water bath. To this cooled solution was added drop wise in 30 min in total 51 ml (38.91 mmol, 1.02 eq) of the Grignard solution of example 16, keeping the inside temperature below 22°C. The reaction mixture was cooled in ice and 25 ml water was added slowly. The formed slurry was filtered and the precipitate was washed with 50 ml ethyl acetate. The combined filtrates were washed with water (50 ml and 100 ml) and brine (100 ml), dried on Na₂SO₄, filtered, and evaporated under vacuum to yield 17.19 g crude product as an oil. This was purified by column chromatography to giving compound according to formula (4a) as pure diastereoisomer.

### Preparative EXAMPLE 18

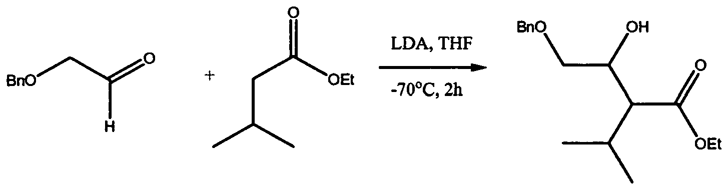

Diisopropylamine (3.56 mL, 25.3 mmol, 1.05 eq.) was dissolved in THF (14 mL) and cooled to -70 °C. *n*-BuLi (1.6 M in hexanes, 15.2 mL, 24.2 mmol, 1.0 eq.) was added dropwise at -70 °C and the resulting yellow solution was stirred at 0 °C for 30 minutes. The solution was then cooled to -70 °C, after which a solution of ethyl isovalerate (3.65 mL, 24.2 mmol, 1.0 eq.) in THF (15 mL) was added drop wise over 20 minutes. The solution was stirred for 1 hour at -70 °C and then benzyloxyacetoaldehyde (3.74 mL, 26.6 mmol, 1.1 eq.) was added drop wise. Subsequently, the reaction was stirred for 3 hours at -70 °C and then allowed to reach room temperature while stirring overnight. The reaction was quenched with NH₄Cl (sat. aq.) and the aqueous layer was extracted with EtOAc (3x). The combined organic layers were washed with brine (sat. aq.) dried (Na₂SO₄), filtered and concentrated *in vacuo* to give the desired products (4.9 g, 17.5 mmol, 72%, mixture of diastereomers) as a yellow oil which was used in the next step without further purification.
¹H-NMR (CDCl₃, 300 MHz) δ = 0.98 (m, 6H, 2 CH*CH*₃), 1.24 (t, 3H, OCH₂*CH*₃), 2.08-2.35 (m, 1H), 2.60 (m, 1H), 3.52 (m, 2H, BnO*CH*2), 4.11 (m, 3H, C3-H and O*CH*₂CH₃ ), 4.54 (m, 2H, Ph*CH*2), 7.32 (m, 5H, Ph) ppm.

### Preparative EXAMPLE 19

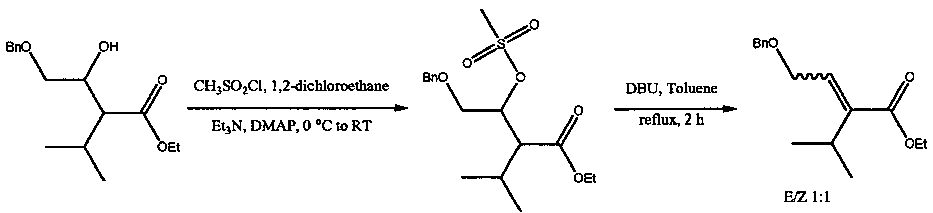

To a solution of the alcohol compound of example 18 (1.0 g, 3.56 mmol) in 1,2-dichloroethane and triethylamine (5.0 mL, 1:1 v/v) at 0 °C was added *N,N-*4-dimethylaminopyridine (44 mg, 0.36 mmol, 10 mol%) and then methanesulfonylchloride (0.83 mL, 10.7 mmol, 3.0 eq.) in a dropwise fashion. The resulting solution was stirred for 48 hours while warming to room temperature. The reaction mixture was quenched with NH₄Cl (sat. aq.) and diluted with water. The layers were separated and the aqueous layer was extracted with chloroform (3x). The combined organic layers were washed with NaHCO₃ (sat. aq.) and brine (sat. aq.), dried (Na₂SO₄), filtered and concentrated *in vacuo* to give the mesylated product as a yellow/brown oil, which was used in the next step without further purification.

The mesylated compound was dissolved in toluene (10 mL) and diazabicyclo-undecene (DBU, 0.64 mL, 4.3 mmol, 1.2 eq.) was added. The resulting solution was heated under refluxing conditions for two hours under a nitrogen atmosphere. The reaction mixture was allowed to cool to room temperature and diluted with EtOAc. Subsequently, the organic solution was washed with NH₄Cl (sat. aq.) and brine (sat. aq.), dried (Na₂SO₄), filtered and concentrated *in vacuo.* The residue was purified by column chromatography (heptane-EtOAc 95:5 v/v) giving 0.88 g of product (3.36 mmol, 94% over 2 steps, 1:1 E/Z-mixture) as a yellow oil.
¹H-NMR of product (1:1 E/Z mixture) (CDCl₃, 300 MHz) δ = 1.09 (d, *J* = 6.6 Hz, 3H, CH*CH*₃),1.18 (d, *J* = 6.9 Hz, 3H, CH*CH*₃),1.30 (t, 3H, OCH₂*CH*₃),2.77 (m, 1H, *CH*(CH₃)₂), 4.12-4.26 (m, 3H, C4-H, O*CH*₂CH₃), 4.41 (m, 1H, C4-H), 4.55 (m, 2H, Ph *CH*2), 6.01 (dt, *J* = 1.2, 5.1 Hz, 1H, C3-H), 6.69 (t, *J* = 5.7 Hz, 1H, C3-H), 7.34 (m, 5H, Ph) ppm.

### Preparative EXAMPLE 20

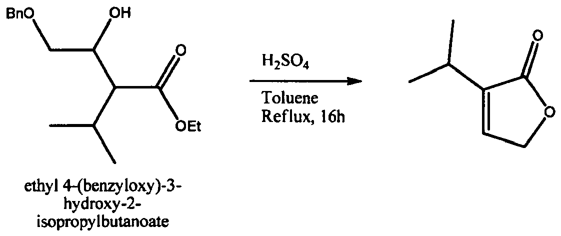

Ethyl 4-(benzyloxy)-3-hydroxy-2-isopropylbutanoate (3.9 g, 14 mmol) was dissolved in toluene (25 mL) and the resulting solution was heated under reflux overnight in the presence of stoichiometric sulfuric acid (96%, 0.74 mL, 14 mmol). The reaction mixture was allowed to cool to room temperature after which the reaction was quenched with NaHCO₃ (sat. aq.). The aqueous layer was extracted with chloroform (3x) and the combined organic layers were dried (Na₂SO₄), filtered and concentrated *in vacuo.* Subsequently, the product was diluted with pentane and extracted with acetonitrile. The acetonitrile layer was washed with pentane (2x) and then concentrated *in vacuo.* The product was further purified by column chromatography (heptane-EtOAc 4:1 v/v) and Kugelrohr distillation to give the unsaturated lactone (1.3 g, 10 mmol, 74%) as a colorless liquid.
¹H-NMR (CDCl₃, 300 MHz) δ = 1.19 (d, *J* = 6.9 Hz, 6H, 2 CH₃), 2.69 (m, 1H, *CH*(CH₃)₂), 4.76 (m, 2H, C4-H₂), 7.07 (m, 1H, C3-H) ppm.

### Preparative EXAMPLE 21

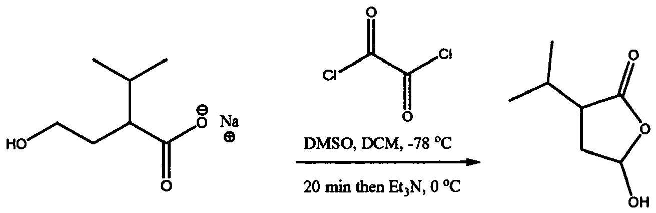

A solution of DMSO (0.41 ml, 5.8 mmol, 3.8 eq.) in DCM (7.5 mL) was added to a solution of oxalylchloride (0.24 ml, 2.9 mmol, 1.9 eq.) in DCM (7.5 mL) at -78 °C under a nitrogen atmosphere. The resulting solution was stirred at -78 °C for 20 minutes after which first 250 mg of the sodium salt depicte din the equation (1.49 mmol) in DCM (7.5 mL) and then acetic acid (0.20 mL, 3.5 mmol, 2.3 eq.) were added. The resulting suspension was stirred for another 20 minutes at -78 °C after which triethylamine (2.5 mL, 18 mmol, 12 eq.) was added. The reaction mixture was allowed to reach 0 °C and stirred at this temperature for 4 hours and then at room temperature overnight. The reaction was quenched with water and the pH of the aqueous layer was adjusted to pH 3-4 with HCl (1 M, aq.). The aqueous layer was extracted with DCM (4x) and the combined organic layers were dried (Na₂SO₄), filtered and concentrated *in vacuo*. ¹H-NMR of the crude product showed a mixture of products. The desired hemiacetal was identified by a characteristic peak at 5.77 ppm (C4-H). Importantly, no overoxidation to the di-carboxylic acid or the corresponding anhydride was observed. The presence of the hemiacetal was confirmed by GC-MS: m/z = 144.

### PREPARATIVE EXAMPLE 22

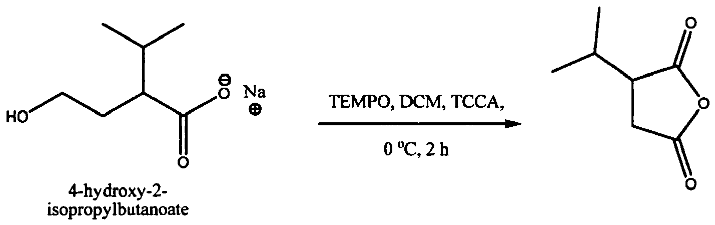

TEMPO (1.8 mg, 12 µmol, 2 mol%) was added to a suspension of the sodium salt of 4-hydroxy-2-isopropylbutanoate (100 mg, 0.60 mmol) in DCM (5.0 mL) at 0 °C. Subsequently, trichlorocyanuric acid (152 mg, 0.65 mmol, 1.1 eq.) was added upon which the suspension turned yellow. The reaction mixture was stirred at 0 °C for 2 hours and then diluted with water. The organic layer was dried (Na₂SO₄), filtered and concentrated *in vacuo* to give the anhydride as the sole product.
¹H-NMR (CDCl₃, 300 MHz) δ = 0.99 (d, *J* = 6.9 Hz, 3H, CH₃), 1.04 (d, *J* = 6.9 Hz, 3H, CH₃), 2.30 (m, 1H, *CH*(CH₃)₂), 4.76 (dd, *J* = 5.4, 18.6 Hz, 1H, C2-H), 3.05 (m, 2H, C3-H₂) ppm.

### Preparative EXAMPLE 23

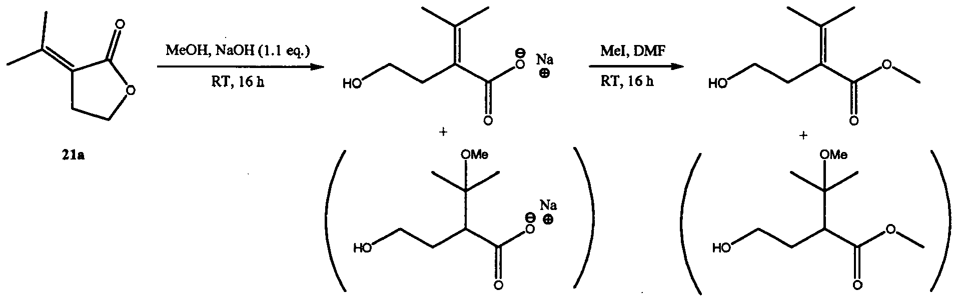

Unsaturated lactone **21a** (5.0 g, 40 mmol) was dissolved in methanol (50 mL) and NaOH (1.8 g, 44 mmol, 1.1 eq.) was added. The resulting solution was stirred overnight at room temperature and then concentrated *in vacuo* to give the desired ring-opened salt as an off-white solid. ¹H-NMR shows that the desired compound was contaminated with a side product (approximately 20%) which was identified as the salt depicted in the equation formed by 1,4-addition of methanol
¹H-NMR of desired salt (CD3OD, 300 MHz) δ = 1.70 (s, 3H, CH₃), 1.83 (s, 3H, CH₃), 2.49 (t, *J* = 6.6 Hz, 2H, C3-H₂), 3.63 (t, *J* = 6.6 Hz, 2H, C4-H₂) ppm.
¹H-NMR of side product (CD3OD, 300 MHz) δ = 1.25 (s, 3H, CH₃), 1.28 (s, 3H, CH₃), 2.49 (m, 2H, C3-H₂), 2.60 (dd, *J* = 3.0, 11.4 Hz, 1H, C2-H), 3.21 (s, 3H, CH₃O), 3.60 (t, 2H, C4-H₂) ppm.

The mixture of salts obtained above (4:1 mol/mol, 720 mg, 4.2 mmol) was dissolved in DMF (3.0 mL) and Mel (0.32 mmol, 5.0 mmol, 1.2 eq.) was added. The resulting mixture was stirred overnight at room temperature under a nitrogen atmosphere. The reaction was quenched with water and the resulting homogeneous mixture was extracted with chloroform (2x). The combined organic layers were dried (Na₂SO₄), filtered and concentrated *in vacuo.* Residual DMF was removed by coevaporation with toluene to give a mixture of the desired ester and the ester derived from the side-product (4:1 mol/mol, 600 mg, 3.6 mmol, 87%) as a yellow oil.
¹H-NMR of desired ester (CDCl₃, 300 MHz) δ = 1.79 (s, 3H, CH₃), 1.93 (s, 3H, CH₃), 2.51 (t, *J* = 6.3 Hz, 2H, C3-H₂), 3.62 (m, 2H, C4-H₂), 3.67 (s, 3H, OCH₃) ppm.
¹H-NMR of ester of side product (CDCl₃, 300 MHz) δ = 1.14 (s, 3H, CH₃), 1.16 (s, 3H, CH₃), 2.51 (m, 2H, C3-H₂), 2.74 (dd, *J* = 3.3, 10.5 Hz, 1H, C2-H), 3.14 (s, 3H, CH₃O), 3.60 (t, 2H, C4-H₂), 3.67 (s, 3H, C(O)OCH₃) ppm.

### EXAMPLE 24

### Preparation of a compound according to formula (7)

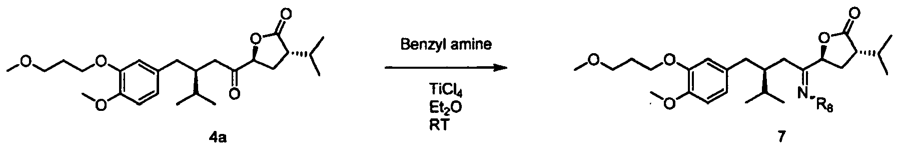

A solution of the compound according to formula (4a) with R₄=2-propyl (868 mg) and benzyl amine (1.07 g) in diethyl ether (4.4 mL) was degassed with N₂. To the solution, a 1 M solution of TiCl₄ in toluene (1.1 mL) was slowly added under vigorous stirring. After stirring for 1 h at room temperature, the heterogeneous reaction mixture was diluted with diethyl ether (5.4 mL) and filtrated. Extraction of the filtrate by 0.5 N NaOH (2 x 50 mL), drying the solution over Na₂SO₄ anhydrous, filtration and subsequent evaporation gave the corresponding imine, a compound according to formula (7) (R₈=benzyl) in 0.8 g yield.
GCMS: M/z: 524.

### EXAMPLE 25-27

### Hydrogenation experiments of compound according to formula (7). Preparations of a compound according to formula (2)

### Method A

The imine compound as obtained in example 24 (0.052 g) was dissolved in a solvent (5 mL). [Ir(COD)Cl]₂ (0.0016 g) was added and dissolved. After preconditioning of the homogeneous catalyst mixture by 5 cycles of N₂ (3 bar) and by 5 cycles of H₂ (25 bar), the solution was stirred for 3 h at 50°C and 25 bar H₂.

### Method B

A solution of benzyl amine (0.021 g) in CH₂Cl₂ (5 mL) was prepared. Under an atmosphere of N₂, [Ir(COD)Cl]₂ (0.0034 g) was dissolved in the benzyl amine solution (1 mL) giving a catalyst solution. The catalyst solution (0.1 mL) was placed in a reaction vessel and the CH₂Cl₂ removed in vacuo and the remaining complex subsequently dissolved in 5 mL of a 0.01 M methanol solution of the imine compound as obtained in example 24. After preconditioning of the methanol reaction mixture by 5 cycles of N₂ (3 bar) and by 5 cycles of H₂ (25 bar), the solution was stirred for 3 h at 50°C and 25 bar H₂.

### Results:

| Exp. | Method | Solvent | Product (area %) | |
|---|---|---|---|---|
| | | | (4S,5S)-2 | (4S,5R)-2 |
| 25 | A | Isopropanol | 52 | 40 |
| 26 | A | Ethyl acetate | 53 | 28 |
| 27 | B | Methanol | 69 | 23 |

| | | | | |
|---|---|---|---|---|
| Up to ca 10% of the undesired 4(R)-2 stereoisomers are also detected. | | | | |

### EXAMPLE 28

### Preparation of a compound according to formula (2) from the compound according to formula (4) - 2 steps.

A solution of ketone (compound according to formula (4a) with R₄=2-propyl) (359 mg) and benzyl amine (443 mg) in diethyl ether (2 mL) was degassed by N₂. Then, (0.5 mL) of 1 M solution of TiCl₄ in toluene was slowly added at room temperature and the heterogeneous mixture was allowed to stir for 0.1 hour. The reaction mixture subsequently was diluted by diethyl ether (5 mL) followed by filtration of the precipitates and the residue washed with 5 portions of 1 mL diethyl ether. According to GC analysis, the keton was completely converted to the imine and the excess of benzyl amine remains in the diethyl ether solution. Due to evaporation of diethyl ether during the filtration step, the total volume of the imine solution was adjusted with diethyl ether to a total amount of 10 mL.

For hydrogenation, (2.5 mL) of the diethyl ether solution of the imine solution was placed in a vessel, and the diethyl ether was evaporated by a stream of N₂. The residue was subsequently dissolved in methanol (10 mL). Meanwhile, [Ir(COD)Cl]₂ (0.0024 g) was dissolved in a solution of a 0.03 M solution of benzyl amine in CH₂Cl₂ (1 mL) and allowed to stand for 0.25 h. After removal of the CH₂Cl₂ by a stream of N₂, the remaining complex was dissolved in the previously prepared solution of the imine in methanol (10 mL) and subsequently the reaction mixture was subjected to hydrogenation. Prior to hydrogenation, the reaction mixture was degassed by 5 cycles of N₂ (3 bar) and by 5 cycles of H₂ (25 bar) and the hydrogenation was conducted for 3 h at 50°C and 25 bar H₂.

After hydrogenation, the reaction mixture was filtrated and treated with 1 N HCl (1 mL) overnight. The methanol was removed in vacuo and the residue dissolved in isopropanol (10 mL) followed by removal of water by azeotropic distillation to dryness. The residue was dissolved in a 1:1 mixture of heptane and ethyl acetate, filtrated over SiO₂ and the SiO₂ washed by the 1:1 heptane/ethyl acetate mixture (3 x 15 mL). Then, the SiO₂ was washed by methanol giving the HCl-salt solution in methanol. The methanol was distilled and the residue dissolved in a mixture of CH₂Cl₂ (2 mL) and triethylamine (0.2 mL) followed by extraction with sat. NaHCO₃ (2 x 1 mL). The CH₂Cl₂ was dried over Na₂SO₄ anhydrous, filtrated, followed by removal of CH₂Cl₂ by distillation. According to GC, the product was obtained in 60 area % of the 4(S), 5(S)-stereoisomer (R₈=benzyl).

### EXAMPLE 29

### Preparation of a compound according to formula (2) from the compound according to formula (4) Reductive amination protocol - 1-pot.

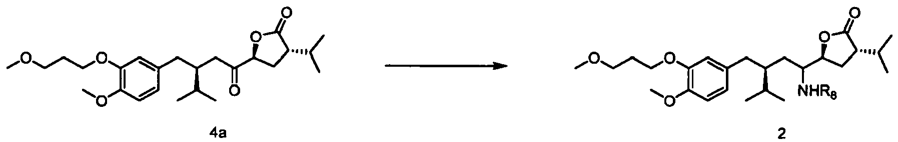

Placed in a reaction vessel, [((S)-tol-BINAP)RuCl₂ (DMF)ₓ] (10 mg), ketone according to formula (4a) (40 mg) ammonium formate (189 mg) and dissolved in 7 N NH₃ in methanol. The reaction mixture was degassed by N₂ and heated at 85 °C. After 2 hours, a sample of the reaction mixture was analysed by GC and the formation of product (4S,5S)-2 (R₈ = H) has been demonstrated in comparison to a standard.

### EXAMPLE 30

### Preparation of a compound according to formula (2) from the compound according to formula (7) using NaBH₄ as reducing agent.

A sample of the imine obtained as in example 24, was dissolved in methanol, treated with excess of NaBH₄ for 1 h and subsequently quenched by 6 N HCl. The methanol was removed in vacuo and the residue dissolved in water. After basifying the water layer to pH 11 with 32 % NaOH in water, the product was extracted by CH₂Cl₂. According to GC, the product was obtained in 60 area % of the 4(S), 5(S)-stereoisomer of compound according to formula (2) with (R₈ = benzyl).

### EXAMPLE 31

### Racemisation of the 4-(S) center of the keton according to formula (4a)

A solution of (4S)-4a with R₄=2-propyl (2.17 g) in isopropanol (20 mL) was stirred for 2 h at 70°C in the presence of KHCO₃ (0.5 g). The reaction mixture was cooled to room temperature and isopropanol removed in vacuo at 40°C and the residue dissolved in CH₂Cl₂ (20 mL). Extraction of the CH₂Cl₂ solution with water (3 x 50 mL), dried over Na₂SO₄ anhydrous, filtration and evaporation of the solvent gave a viscous oil. Analysis showed full racemisation of the 4-C stereogenic center, while the other stereocenters (2-(S) and 7-(S)) are still optically pure.

### EXAMPLE 32

### Preparation of a compound according to formula (7) using (R)-α-methyl benzyl amine.

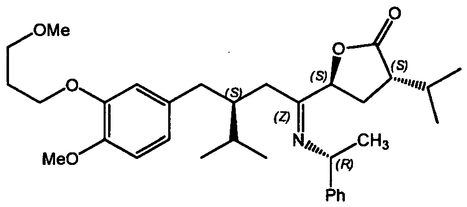

A solution of ketone 4a (178 mg) and (R)-α-methy) benzyl amine (264 µL) in ether (2 mL) was degassed by N₂. To the solution, a 1 M solution of TiCl₄ in toluene (0.25 mL) was slowly added under vigorous stirring. The reaction mixture was allowed to stir for 2 hours, filtrated and the residue washed extensively with excessive amounts of ether. In order to remove residual amounts of α-methyl benzyl amine the ether solution was extracted with diluted NaHCO₃ (3 x 5 mL) and water (5 mL). The ether layer was separated, dried over Na₂SO₄ anhydrous, and concentrated. The residue was used as such in the subsequent reactions.

### EXAMPLE 33

### Preparation of a compound according to formula (7) using (S)-α-methyl benzyl amine.

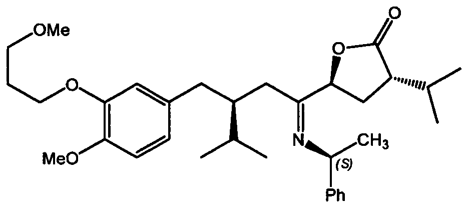

A solution of ketone 4a (178 mg) and (S)-α-methyl benzyl amine (264 µL) in ether (2 mL) was degassed by N₂. To the solution, a 1 M solution of TiCl₄ in toluene (0.25 mL) was slowly added under vigorous stirring. The reaction mixture was allowed to stir for 2 hours, filtrated and the residue washed extensively with excessive amounts of ether. In order to remove residual amounts of α-methyl benzyl amine the ether solution was extracted with diluted NaHCO₃ (3 x 5 mL) and water (5 mL). The ether layer was separated, dried over Na₂SO₄ anhydrous, and concentrated. The residue was used as such in the subsequent reactions.

### EXAMPLE 34

### Preparation of a compound according to formula (7) using (R)-α-methyl benzyl amine and the ketone of formula 4a with the 4-C center being racemic.

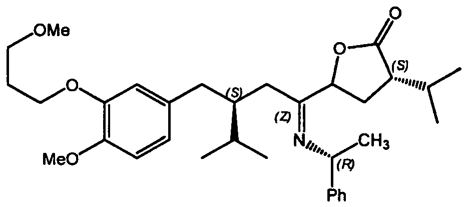

A solution of ketone 4a (178 mg) and (R)-α-methyl benzyl amine (264 µL) in ether (2 mL) was degassed by N₂. To the solution, a 1 M solution of TiCl₄ in toluene (0.25 mL) was slowly added under vigorous stirring. The reaction mixture was allowed to stir for 2 hours, filtrated and the residue washed extensively with excessive amounts of ether. In order to remove residual amounts of α-methyl benzyl amine the ether solution was extracted with diluted NaHCO₃ (3 x 5 mL) and water (5 mL). The ether layer was separated, dried over Na₂SO₄ anhydrous, and concentrated. The residue was used as such in the subsequent reactions.

### EXAMPLE 35

### Preparation of a compound according to formula (7) using (S)-α-methyl benzyl amine and the ketone of formula 4a with the 4-C center being racemic.

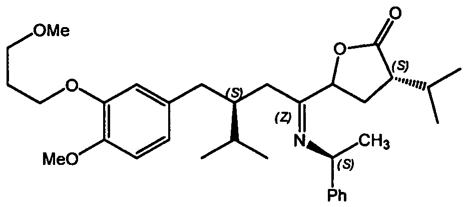

A solution of ketone 4a (178 mg) and (S)-α-methyl benzyl amine (264 µL) in ether (2 mL) was degassed by N₂. To the solution, a 1 M solution of TiCl₄ in toluene (0.25 mL) was slowly added under vigorous stirring. The reaction mixture was allowed to stir for 2 hours, filtrated and the residue washed extensively with excessive amounts of ether. In order to remove residual amounts of α-methyl benzyl amine the ether solution was extracted with diluted NaHCO₃ (3 x 5 mL) and water (5 mL). The ether layer was separated, dried over Na₂SO₄ anhydrous, and concentrated. The residue was used as such in the subsequent reactions.

### EXAMPLE 36 - 39

### Reduction of the imines of examples 32-35 using NaBH4, and subsequent hydrogenolysis (preparation of compound according to formula 2 with R₈ = H)

Methanol (5 mL) was added to the residue of example 32, 33, 34, and 35. In each vessel NaBH₄ (40 mg) was added and after complete conversion, the reaction mixtures were quenched with 1 N HCl (1 mL), the solvents removed in vacuo and water (2 mL) added to the residue. In the presence of CH₂Cl₂ (2 mL), the pH was adjusted to 13 with 32 % NaOH under vigorous stirring and cooling. The CH₂Cl₂ was separated and the water layer extracted with CH₂Cl₂ (1 mL). The combined CH₂Cl₂ layers dried over Na₂SO₄ anhydrous, filtrated and the CH₂Cl₂ removed.

### Hydrogenolysis

For this purpose, 45 mg of the above obtained amines were dissolved in methanol (5 mL). The reaction mixture was treated by 5 cycles of N₂ (3 bar) and 5 cycles of H₂ (25 bar) in the presence of wet 10 % Pd/C (80 mg). The hydrogenolysis subsequently was performed at 50°C and 25 bar of H₂ for 2 hours. Analysis of the reaction mixtures by GC, see table below.

| Example | Imine | Product (area %) | | |
|---|---|---|---|---|
| | | (4S,5S)-2 | (4S,5R)-2 | (4R)-2 |
| 36 | Example 32 | 14 | 80 | 6.5 |
| 37 | Example 33 | 75 | 12 | 13 |
| 38 | Example 34 | 15 | 39 | 46 |
| 39 | Example 35 | 46 | 7 | 46 |

### EXAMPLE 40 - 43

### Synthesis of compounds according to formula 2 (R₈ = α-methyl benzyl) by preparing the imine of compound 4a and α-methyl benzyl amine and subsequent reduction using different hydrides_{.}

To a solution of (4S)-4 (2.17 g) in THF ( 8 mL) was respectively added α-methyl benzyl amine (1.45 g) and Ti(O*ⁱ*Pr)₄ (2.98 g) and the mixture was allowed to stir overnight. A fraction of the obtained solution (1.37 g) was placed in a 5 mL vial, degassed by N₂ and subsequently a 1 eq. of the hydride source was introduced. The mixture was allowed to stir for 40 hours at room temperature. For HPLC analysis, a sample was prepared by hydrolysis of the reaction mixture (0.2 mL) in 1 N HCl (0.3 mL). Extraction was carried out by a mixture of CH₂Cl₂ (1 mL) and triethylamine (0.1 mL) in the presence of an additional amount of water (1 ml). The CH₂Cl₂ layer was separated and dried over Na₂SO₄ anhydrous, filtrated and the volatiles removed giving a residue containing the product. The obtained residue was dissolved in the HPLC-eluent and the solution subjected to HPLC-analysis, see table below.

| Ex. | [H⁻] | Product | Product (area %) | | |
|---|---|---|---|---|---|
| | | | (4S,5S) | (4S,5R) | (4R) |
| 40 | 2M LiBH₄ in THF | 2 (R₈ = (R)-α-methy) benzyl) | 82 | 12 | 6 |
| 41 | NaCNBH₃ | 2 (R₈ = (R)-α-methyl benzyl) | 73 | 25 | 2 |
| 42 | NBu₄BH₄ | 2 (R₈ = (S)-α-methyl benzyl) | 72 | 21 | 7 |
| 43 | BH₃ | 2 (R₈ = (R)-α-methyl benzyl) | 22 | 72 | 5 |

### EXAMPLE 44 - 47

### Synthesis of compounds according to formula 2 (R₈=(R)-α-methyl benzyl) by preparing the imine of compound 4a and (R)-α-methyl benzyl amine and subsequent hydrogenation using PVC in different solvents.

(R)-α-methyl) benzyl amine (0.66 g) and Ti(O*ⁱ*Pr)₄ (2.99 g) was slowly added to a solution of (4S)-4 (2.17 g) in dry THF (6.4 mL) and the mixture was allowed to stir for 40 hours at room temperature. The reaction mixture was diluted with a solution of water (1 mL) in THF (50 mL) and after stirring for 1 night the solids were filtrated. Then removal of THF in vacuo at 30°C and the residue was immediately dissolved in EtOAc (35 mL) followed by azeotropic distillation of water giving the crude product in 2.48 g yield. The crude product was dissolved in MTBE (10mL) and the obtained solution used as such in hydrogenation reactions.

For hydrogenations, the substrate solution in MTBE (1.25 mL) was evaporated to dryness and dissolved in a solvent (5 mL). To the obtained substrate solution, 5 % Pt/C wet (140 mg) was added and the mixture prepared for hydrogenation by 5 cycles of N₂ (3 bar) and 5 cycles of H₂ (25 bar). The hydrogenations were carried out at room temperature and 25 bar H₂ for 24 hours. According to GC, complete conversion of the imine-derivative was achieved. The stereomeric ratios of product 2 (R₈ = (R)-α-methyl benzyl amine) was determined by HPLC, see table below.

| Example | Solvent | Product 2 (R₈ = (R)-α-methyl benzyl) (area %) | | |
|---|---|---|---|---|
| | | (4S,5S) | (4S,5R) | (4R) |
| 44 | THF | 26 | 61 | 12 |
| 45 | IPA | 20 | 66 | 13 |
| 46 | MTBE | 22 | 66 | 12 |
| 47 | EtOAc | 32 | 55 | 13 |

### EXAMPLE 48 and 49

### Synthesis of compounds according to formula 2 (R₈ = (R)-α-methyl benzyl) by preparing the imine of compound 4a and (R)-α-methyl benzyl amine and subsequent hydrogenation using Pt/C in different solvents.

(R)-α-methyl benzyl amine (1.21 g) and Ti(O*ⁱ*Pr)₄ (5.98 g) were slowly added to a solution of (4S)-4 (4.34 g) in dry THF (16 mL) and the mixture was allowed to stir for 40 hours at room temperature.

For hydrogenation, the imine-derivative solution in THF (1 mL) was placed in a reaction vessel and after removal of the solvent the residue was dissolved in a solvent (5 mL), then addition of Pt/C wet (140 mg) and the mixture prepared for hydrogenation by 5 cycles of N₂ (3 bar) and 5 cycles of H₂ (25 bar). The hydrogenations were carried out at 50°C and 25 bar H₂ overnight. According to GC, complete conversion of the imine-derivative was achieved. The stereomeric ratios of compound according to formula 2 (R₈ = (R-α-methyl benzyl amine) was determined by H PLC, see table below

| Example | solvent | Product 2 (R₈ = (R)-α-methyl benzyl amine) (area %) | | |
|---|---|---|---|---|
| | | (4S,5S) | (4S,5R) | (4R) |
| 48 | MeOH | 25 | 61 | 15 |
| 49 | IPA | 24 | 61 | 15 |

### EXAMPLE 50 and 51

### Synthesis of compounds according to formula 2 (R₈ = H) by preparing the imine of compound 4a and α-methyl benzyl amine and subsequent hydrogenation using Pd/C.

A solution of ketone (4S)-4 (1.09 g), the α-methyl benzyl amine (363 mg) and triethyl amine (1.01 g) in ether (12 mL) was degassed by N₂. Then, slow addition of 1 M of TiCl₄ in toluene (1.5 mL) at room temperature and the mixture allowed to stir for 3 hour. From the obtained reaction mixture, 1.2 mL was diluted by ether (2 mL) and extracted with a NaHCO₃ solution in water (3 x 1 mL), the ether layer separated, dried over Na₂SO₄, filtrated and the ether removed. The obtained residue was dissolved in methanol and the obtained solution prepared for hydrogenation by 5 cycles of N₂ (3 bar) and 5 cycles of H₂ (25 bar). The hydrogenation was carried out in the presence of wet 10 % Pd/C (80 mg) at 50°C and 25 bar of H₂ for 2 hours. After hydrogenation, a sample was filtrated and the stereomeric ratios of compound according to formula 2 (R₈ = H) was determined by GC-analysis, see table below

| Example | amine | Product 2 (R₈=) H (area %) | | |
|---|---|---|---|---|
| | | (4S,5S) | (4S,5R) | (4R) |
| 50 | (R)-α-Methyl benzyl amine | 95 | 5 | |
| 51 | (S)-α-Methyl benzyl amine | 63 | 32 | 5 |

### EXAMPLE 52

### Synthesis of compound according to formula 2 (R₈ = H) by preparing the imine of compound 4a and (R)-α-methyl benzyl amine and subsequent hydrogenation using Pd/C in isopropanol.

A solution of ketone (4S)-4 (434 mg) in THF (1.6 mL) was degassed by N₂. Then slow addition of (R)-α-methyl benzyl amine (308 µL) and Ti(O*ⁱ*Pr)₄ (0.63 mL) and the reaction mixture allowed to stir for 24 hours.

The imine product was isolated by means of column chromatography. For this purpose, a column was charged with SiO₂ and heptane (10 mL). In the clear heptane phase, 1 mL of the reaction mixture was dissolved and eluated on top of the SiO₂. The eluation was continued by a mixture of EtOAC/heptane and 3 fractions of 10 mL were collected. Fraction 1 and 2 were combined and residual amounts of TiO₂ filtrated following by removal of the solvent in vacuo at 30°C. The residue (0.14 g) was dissolved in isopropanol and according to GC-analysis, the solution was free from (R)-α-Methyl benzyl amine.

For hydrogenation, 5 mL of the isopropanol solution was placed in a reaction vessel and conditioned by 5 cycles of N₂ (3 bar) and 5 cycles of H₂ (25 bar). The hydrogenation was conducted in the presence of dry 5 % Pd/C (20 mg) at 50°C and 25 bar of H₂ for 20 hours giving the compound according to formula 2 (R₈ = H) in stereomeric ratios of (4S,5S)-2 (74 area %), (4S,5R)-2 (6 area %), (4R)-2 (20 area %) according to GC-analysis.

### EXAMPLE 53

### Synthesis of compound according to formula 2 (R₈ = H) by preparing the imine of compound 4a and (R)-α-methyl benzyl amine and subsequent hydrogenation using Pd/C in MTBE.

(R)-α-methyl benzyl amine (1.30 g) and Ti(O*ⁱ*Pr)₄ (5.98 g) were slowly added to a solution of ketone (4S)-4 (4.34 g) in dry THF (13 mL) and the mixture was allowed to stir for 40 hours at room temperature. The reaction mixture was slowly added to a vigorous stirring solution of water (2 mL) and triethyl amine (305 mg) in THF (100 mL) and after 0.75 hours the reaction mixture was filtrated. The volatiles were removed in vacuo at 30°C and the obtained residue dissolved in EtOAc (85 mL) followed by azeotropic distillation of residual amounts of water under reduced pressure at 30°C. The obtained residue was dissolved in MTBE (20 mL).

For hydrogenation, the MTBE solution (1 mL) was placed in a reaction vessel and the MTBE removed. The obtained residue was dissolved in THF (5 mL) followed by the addition of dry 5 % Pd/C (200 mg). After conditioning the solution by 5 cycles of N₂ (3 bar) and 5 cycles of H₂ (25 bar), the reaction mixture was subjected to hydrogenation overnight at 50°C and 25 bar of H₂ giving compound according to formula 2 (R₈ = H) in stereomeric ratios of (4S,5S)-2 (72 area %), (4S,5R)-2 (9.7 area %), (4R)-2 (18.5 area %) according to HPLC-analysis.

### EXAMPLE 54

### Synthesis of compound according to formula 2 (R₈ = H) by preparing the imine of compound 4a and (R)-α-methyl benzyl amine and subsequent hydrogenation using Pd/C in THF.

Stock solution of imine compound: A solution of the imine compound was prepared by slow addition of (R)-α-methy) benzyl amine (3.63 g) and Ti(O*ⁱ*Pr)₄ (9.38 g) to a solution of (4S)-4 (13.02 g) in dry THF (50 mL). The mixture was allowed to stir at room temperature and monitored by GC-analysis until complete conversion.

1.18 g of this imine solution was treated with water (20.6 mg) for 0.25 h at 50°C and the precipitate removed by filtration at room temperature. The clear THF solution was diluted by THF to a total volume of 5 mL and after addition of dry 5 % Pd/C (200 mg) conditioned for hydrogenation by 5 cycles of N₂ (3 bar) and 5 cycles of H₂ (25 bar). The mixture was subjected to hydrogenation at 50°C and 25 bar of H₂ to full conversion of the starting material giving compound according to formula 2 (R₈ = H) in stereomeric ratios of (4S,5S)-2 (78 area %), (4S,5R)-2 (9.5 area %), (4R)-2 A (11.9 area %) according to HPLC-analysis.

### EXAMPLE 55

### Synthesis of compound according to formula 2 by preparing the imine of compound 4a and (R)-α-methvl benzyl amine and subsequent hydrogenation using Pd/C and LiH as an additive.

1.18 g of the imine solution of example 54 was placed in a reaction vessel and diluted with THF anhydrous (3.3 g) under an atmosphere of N₂. Then, LiH (8 mg) and dry 5 % Pd/C (200 mg) was added and after conditioning by 5 cycles of N₂ (3 bar) and 5 cycles of H₂ (25 bar) the mixture was subjected for hydrogenation at 50°C and 25 bar of H₂ for 20 hours giving, according to GC, a mixture of the compound according to formula 2 (R₈ = H) (23 area %) and compound according to formula 2 (R₈ = (R)-α-methyl benzyl amine) (49 area %). According to HPLC, the optical purity of the compound according to formula 2 (R₈ = H) was as follows: (4S,5S) (90 area %), (4S,5R) (4.8 area %), (4R) (5.5 area %).

### EXAMPLE 56

### Synthesis of compound according to formula 2 by preparing the imine of compound 4a and (R)-α-methyl benzyl amine and subsequent hydrogenation using Pd(OH)₂/C.

11.8 g of the imine solution of example 54 was placed in a reaction vessel and diluted with THF anhydrous (33 g) and heated to 50°C. To the obtained warm solution, water (200 µL) was added and the mixture stirred for 0.25 hours. Then, the mixture was cooled to room temperature followed by filtration of the formed precipitate. From the obtained clear solution, 4.5 g was placed in a reaction vessel containing dry 20 % Pd(OH)₂/C (75 mg). The reaction mixture was degassed by 5 cycles of N₂ (3 bar) and 5 cycles of H₂ (25 bar) and the hydrogenation was carried out at 50°C and 25 bar of H₂ for 38 hours giving complete conversion according to GC. According to HPLC, the compound according to formula 2 (R₈ = H) was obtained in stereomeric ratios of (4S,5S)-2 (53 area %), (4S,5R)-2 (22 area %), (4R)-2 (24 area %).

### EXAMPLE 57

### Large scale synthesis of compound according to formula 2 by preparing the imine of compound 4a and (R)-a-methyl benzyl amine and subsequent hydrogenation using Pd/C.

23.7 g of the imine solution of example 54 was placed in a reaction vessel and diluted with THF (52 g) and heated to 50°C. To the obtained warm solution, sat. NaHCO₃ (400 µL) was added and the mixture stirred for 0.25 hours. To the heterogeneous mixture, Na₂SO₄ anhydrous (10 g) and active carbon (6.9 g) was respectively added under vigorous stirring. Then, the mixture was cooled to room temperature followed by filtration of the reaction mixture under N₂ and the wet cake was washed by THF (4 x 8 mL). The obtained clear solution, was placed in the autoclave containing dry 5 % Pd/C (4 g). The autoclave was degassed by 5 cycles of N₂ (3 bar) and 5 cycles of H₂ (25 bar) and the hydrogenation was carried out at 50°C and 25 bar of H₂ for 15 hours giving complete conversion according to GC.

The product was isolated by filtration of the catalyst and subsequent removal of the solvent under reduced pressure. The residue was treated with 1 N HCl (20 mL) and water (50 mL) and the water layer extracted by heptane (3 x 50 mL). The water layer was separated and the pH was increased to 13 by the addition of 32 % NaOH (- 3 g) in the presence of MTBE (40 mL) under vigorous stirring. The MTBE layer was separated followed by an additional extraction of the remaining water layer with MTBE (40 mL). The combined MTBE layers were dried over Na₂SO₄ anhydrous, filtrated and the MTBE evaporated giving the compound according to formula 2 (R₈ = H) in stereomeric ratios of (4S,5S)-2 (82 area %), (4S,5R)-2 (8.3 area %) and (4R)-2 (10.2 area %) according to GC.

### EXAMPLE 58

### Preparation of compound according to formula 13 with X = NHR₅, obtained by reacting a compound according to formula 2 (R₈ = H) with 3-amino-2,2-dimethylpropanamide.

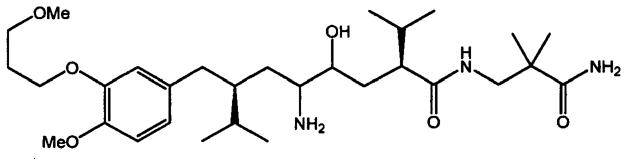

A round bottom flask was charged with compound according to formula 2 (R₈ = H) (8.8 g), 3-amino-2,2-dimethylpropanamide (7.08 g) and triethylamine (3.50 g) and the viscous mixture was stirred at reflux in the presence of MTBE in order to obtain a homogeneous mixture. Under slow heating to 65°C, the MTBE was removed by distillation and the remaining residue allowed to stir for 20 hours at 65°C in the presence of 2-hydroxypyridine (1.94 g). At complete conversion of compound according to formula 2, the reaction mixture was dissolved in MTBE (71 mL) and the MTBE layer extracted by 5 % NaCl in water (2 x 71 mL). The combined water layers were washed by MTBE (3 X 71 mL) and the combined MTBE layers (total of 4 fractions) dried over Na₂SO₄ anhydrous, filtrated and the MTBE evaporated in vacuo giving the crude product in 10.29 g. The crude product was dissolved in MTBE (71 mL) followed by extraction with 1 N HCl (15 mL) and water (2 x 15 mL). The combined water layers washed with MTBE (71 mL) and the combined MTBE layers washed with water (2 x 14 mL). Under cooling and vigorous stirring, the pH of the combined water layers was adjusted to pH 11 by 32 % NaOH in the presence of MTBE (71 mL), MTBE separated and the remaining basic water layer washed with MTBE (5 x 71 mL). The combined MTBE layers were dried over Na₂SO₄ anhydrous, filtrated and removal of MTBE in vacuo affording compound according to formula 13 in with stereomeric ratios of (4S,5S)-13 (71 area %) and other stereoisomers of 13 (29 area %) according to HPLC.

### EXAMPLE 59

### Purification of compound according to formula 13, obtained in example 58, by crystallization with fumaric acid.

A solution of the compound according to formula 13, as obtained in example 58 (1.48 g), and fumaric acid (155.4 mg) in EtOH (15 mL) were heated to 40°C and subsequently the ethanol removed by distillation under reduced pressure to a total weight of 3.21 g. The concentrated ethanol solution was dissolved in CH₃CN (37 mL) at 37°C. The solution slowly was cooled to room temperature and seeded by a few crystals of aliskiren fumarate salt and the mixture was allowed to stir for 19 hours. The precipitated aliskiren fumarate salt was filtrated and dried giving the product.

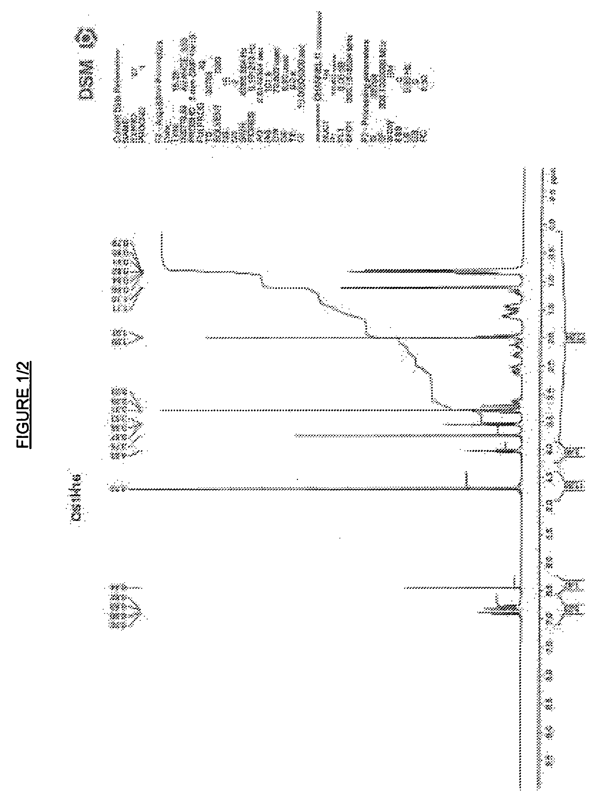

According to ¹H NMR the compound is pure besides a trace of acetonitril left, see Figure 1.

The all (S) purity of the aliskiren fumarate-salt (2.8 g) obtained as such was 93 area % according to HPLC.

### EXAMPLE 60

### Purification of compound according to formula 13, obtained in example 59, by double re-crystallization

The acetonitril containing product 7 fumarate-salt (2.8 g) obtained from first crystallisation was dissolved in ethanol (40 mL) and the total amount reduced to 5.8 g by partial distillation of ethanol. The concentrated ethanol solution was dissolved in CH₃CN (80 mL). The solution was slowly cooled to room temperature and seeded by a few crystals of the aliskiren fumarate-salt and the mixture was allowed to stir for 19 hours. The precipitated aliskiren fumarate-salt was filtrated and dried giving the product in 2.4 g yield in a stereomeric purity of 98.2% of (4S,5S)-Aliskiren fumarate salt.(98.2 area %).

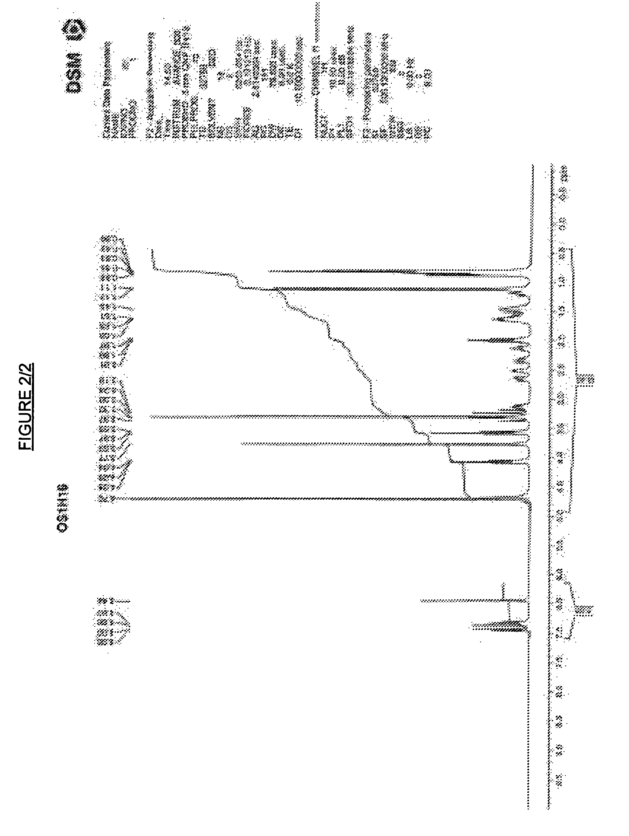

This isolated aliskiren fumarate-salt was redissolved in ethanol and the ethanol partially distilled to a total amount of of the residue of 5.0 g. The obtained residue was dissolved in CH₃CN (80mL) and cooled to room temperature. At room temperature, the solution was seeded by a few crystals of Aliskiren fumarate-salt and the mixture was allowed to stir for 5 hours. The precipitated Aliskiren fumarate-salt was filtrated and dried giving the product in 2.2 g yield in a stereomeric purity of (4S,5S)-7 (98.8 area %), according to HPLC. From the isolated product, a ¹H NMR spectrum was recorded, see Figure 2.

## Claims

1. Process for the preparation of a compound according to formula (13) or its ring-closed form according to formula (2), or a mixture thereof, 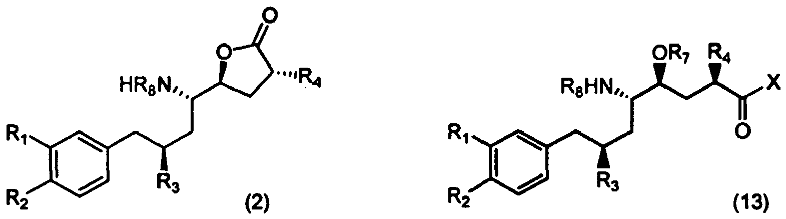
wherein R₁ being selected from the group consisting of F, Cl, Br, I, C₁₋₆halogenalkyl, C₁-₆alkoxy, C₁₋₆alkoxy-C₁₋₆alkyloxy, and C₁₋₆alkoxy-C₁₋₆alkyl;
R₂ being selected from the group consisting of F, Cl, Br,I, C₁₋₄alkyl or C₁₋₄alkoxy;
R₁ and R₂ may be linked together to form a ring structure;
R₃ and R₄ each independently being branched C₃₋₆alkyl;
X stands for NHR₅ or OR₆ wherein
R₅ is C₁₋₁₂cycloalkyl, C₁₋₁₂alkyl, C₁₋₁₂hydroxyalkyl, C₁₋₆alkoxy-C₁₋₆alkyl, C₁₋₆alkanoyloxy-C₁-₆alkyl, C₁₋₁₂aminoalkyl, C₁₋₆alkylamino-C₁₋₆alkyl, C₁₋₆dialkylamino-C₁₋₆alkyl, C₁₋₆alkanoylamino-C₁-₆alkyl, HO-(O)C-C₁₋₁₂alkyl, C₁₋₆alkyl-O-(O)C-C₁₋₆alkyl, H₂N-C(O)-C₁₋₁₂alkyl, C₁₋₆alkyl-HN-C(O)-C₁₋₆alkyl, (C₁₋₆alkyl)₂-N-C(O)-C₁₋₆alkyl; saturated, unsaturated, or partially saturated C₁₋₁₂heterocyclyl bonded via a carbon atom, and which heterocyclyl is optionally substituted one or more times by C₁₋₆alkyl, trifluoromethyl, nitro, amino, *N-*mono- or *N,N*-di-C₁₋₆alkylated amino, C₁₋₆alkanoyl, C₂₋₆alkenyl, C₂₋₆alkynyl, C₁₋₆alkoxy, C₁₋₆alkoxycarbonylamino, C₀₋₆alkylcarbonylamino, C₁₋₆alkylcarbonyloxy. C₁₋₁₂aryl, *N*-mono or *N,N*-di-C₁₋₆alkylated carbamoyl, optionally esterified carboxyl, cyano, halogen, halo-C₁₋₆alkoxy, halo-C₁₋₆alkyl, C₁₋₁₂heteroaryl, saturated, unsaturated or partially saturated C₁₋₆heterocyclyl, hydroxyl, nitro; and R₆ represents H, or optionally substituted C₁₋₁₂alkyl, optionally substituted C₁₋₁₂alkylaryl, or optionally substituted C₁₋₁₂aryl;
R₇ represents H, or is an O-protecting group;
and wherein any two of R₇, R₈, or X are optionally linked together to form a ring structure;
R₈ denotes H; optionally substituted C₁₋₁₂alkyl, optionally substituted C₁₋₁₂alkylaryl, or optionally substituted C₁₋₁₂aryl; optionally substituted C(O)C₁₋₆alkyl; optionally substituted C(O)OC₁₋₆alkyl; optionally substituted C(O)NHC₁₋₆alkyl; or optionally substituted C(O)N(C₁₋₆alkyl)₂; or R₈ denotes
-NHR₉,
-S(O)₂R₉; SOR₉; S(O)₃R₉; S(O)₂N(R₉);
-P(O)(R₉)₂
or R₈ stands for (R₉)₂Y with Y being an anion such as acetate, or halogen; and wherein each R₉ independently represents H, optionally substituted C₁₋₁₂alkyl, optionally substituted C₁₋₁₂alkylaryl, or optionally substituted C₁₋₁₂aryl; comprising the following steps,
a) reacting a compound according to formula (11), or its ring-closed form according to formula (4), or a mixture thereof, 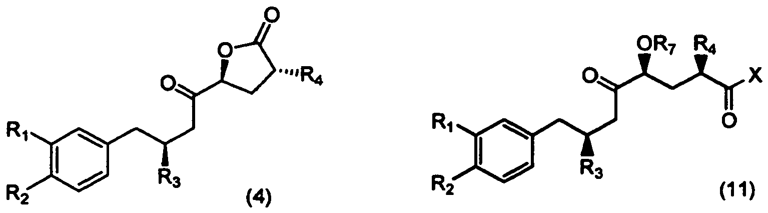 wherein R₁, R₂, R₃, R₄, R₇ and X are each as described above for formula (2) and (13)
with a compound according to formula (5)
R₈-NH₂ (5)
wherein R₈ is as described above for formula (2) and (13) which reaction results in a compound according to formula (7) or a compound according to formula (12) or a mixture thereof, 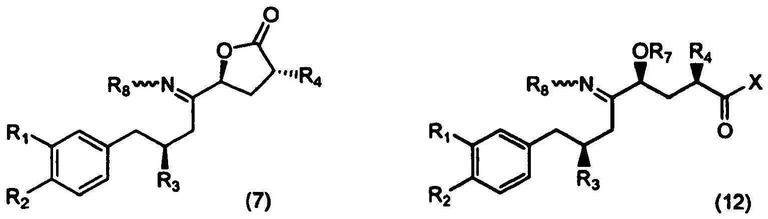 wherein R₁, R₂, R₃, R₄, R₇, R₈ and X are each as described above for formula (2) and (13)
b) further reacting compound according to formula (7) or (12) or a mixture thereof, in the presence of a reducing reagent, and optionally in the presence of a catalyst, and optionally in the presence of one or more additives,
which reaction results in the formation of compound according to formula (2) or formula (13) or a mixture thereof.

2. A process according to claim 1, wherein the compounds according to formula (7) or formula (12) are not isolated from the reaction mixture before carrying out step b).

3. A process according to any one of claims 1-2, wherein step b) is performed in the presence of a catalyst and wherein said catalyst is a transition metal based catalyst.

4. A process according to any one of claims 1-3, comprising reacting the compound according to formula (11), or its ring-closed form according to formula (4), or mixture thereof, with a compound according to formula (5) in the presence of a reducing agent and a catalyst.

5. A process according to claim 4, wherein the reducing agent is selected from the group of molecular hydrogen or a hydrogen donating compound; and wherein when the molecular hydrogen is used it is used in the presence of a transition metal catalyst and when a hydrogen donating compound is used it is used optionally in the presence of a catalyst.

6. A process according to claim 4 or 5 wherein said catalyst is an enzyme, preferably an aminotransferase.

7. A process according to any one of claims 4-6 wherein compound according to formula (5) is also the reducing agent

8. Process according to any one of claims 1-7, wherein X in the compound according to formula (11), and also in resulting compounds according to formula (12), if it is formed, and the compound according to formula (13), respectively, stands for OR₆ and wherein R₆ is as described in Claim 1.

9. Process according to any one of claims 1-7,wherein X in the compound according to formula (11), and also in resulting compounds according to formula (12), if it is formed, and the compound according to formula (13), respectively, stands for NHR₅, and wherein R₅ is as described in Claim 1.

10. Process for the preparation of a compound according to formula (13) or any pharmaceutically acceptable salt thereof, wherein X represent NHR₅, wherein the process comprises a process according to any one of claims 1-9 and wherein R₅ is as described in claim 1.

11. A compound according to formula (11) 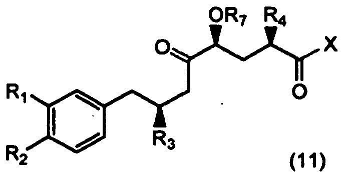 wherein all the R-groups are defined as in Claim 1, and wherein X is selected from the group of OR₆ and NHR₅, and wherein R₆ and R₅ are as described in Claim 1.

12. A compound according to formula (12) 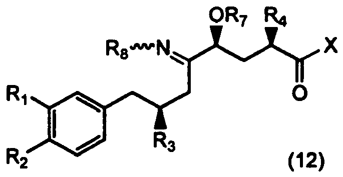 wherein all the R-groups are defined as in Claim 1 and wherein X is selected from the group of OR₆ and NHR₅, and wherein R₆ and R₅ are as described in Claim 1.

13. A compound according to formula (7) 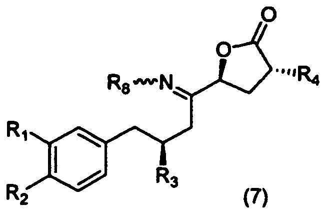 wherein R₁, R₂, R₃, and R₄, are defined as in Claim 1, and wherein R₈ denotes H; optionally substituted C₁₋₁₂alkyl, optionally substituted C₁₋₁₂alkylaryl, or optionally substituted C₁₋₁₂aryl; optionally substituted C(O)C₁₋₆alkyl; optionally substituted C(O)OC₁₋₆alkyl; optionally substituted C(O)NHC₁₋₆alkyl; or optionally substituted C(O)N(C₁₋₆alkyl)₂; or R₈ denotes
-NHR₉,
-S(O)₂R₉; SOR₉; S(O)₃R₉; S(O)₂N(R₉);
-P(O)(R₉)₂;
or R₈ stands for (R₉)₂Y with Y being an anion such as acetate, or halogen; and wherein each R₉ independently represents H, optionally substituted C₁₋₁₂alkyl, optionally substituted C₁₋₁₂alkylaryl, or optionally substituted C₁₋₁₂aryl.

14. Process according to any of claims 1-10, wherein the compound according to formula (2) is further reacted with 3-amino-2,2-dimethylpropanamide to obtain a compound according to formula (13) with X=NHR₅, or pharmaceutically acceptable salts thereof.

15. Process according to any of claims 1-10 and 14, wherein the compound according to formula (2) or formula (13) is further reacted to obtain 2(S),4(S),5(S),7(S)-N-(2-carbamoyl-2-methylpropyl)-5-amino-4-hydroxy-2,7-diisopropyl-8-[4-methoxy-3-(3-methoxypropoxy)fenyl]-octanamide or any pharmaceutically acceptable salt thereof.

## Patentansprüche

1. Verfahren zur Herstellung einer Verbindung gemäß Formel (13) oder ihrer ringgeschlossenen Form gemäß Formel (2) oder eines Gemischs davon, 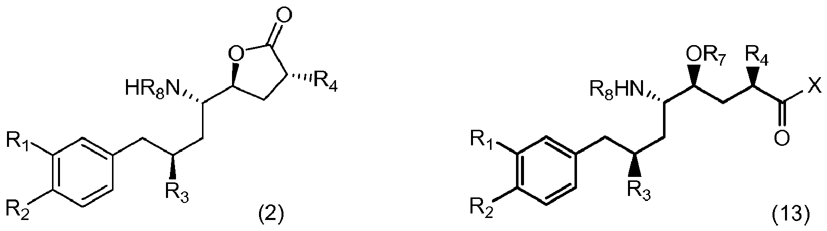
wobei R₁ aus der Gruppe bestehend aus F, Cl, Br, I, C₁-C₆-Halogenalkyl, C₁₋₆-Alkoxy, C₁₋₆-Alkoxy-C₁₋₆-alkyloxy und C₁₋₆-Alkoxy-C₁₋₆-alkyl ausgewählt ist; R₂ aus der Gruppe bestehend aus F, Cl, Br, I, C₁₋₄-Alkyl oder C₁₋₄-Alkoxy ausgewählt ist;
R₁ und R₂ miteinander zu einer Ringstruktur verknüpft sein können;
R₃ und R₄ jeweils unabhängig voneinander für verzweigtes C₃₋₆-Alkyl stehen;
X für NHR₅ oder OR₆ steht, wobei
R₅ für C₁₋₁₂-Cycloalkyl, C₁₋₁₂-Alkyl, C₁₋₁₂-Hydroxyalkyl, C₁₋₆-Alkoxy-C₁₋₆-alkyl, C₁₋₆-Alkanoyloxy-C₁₋₆-alkyl, C₁₋₁₂-Aminoalkyl, C₁₋₆-Alkylamino-C₁₋₆-alkyl, C₁₋₆-Dialkylamino-C₁₋₆-alkyl, C₁₋₆-Alkanoylamino-C₁₋₆-alkyl, HO-(O)C-C₁₋₁₂-Alkyl, C₁₋₆-Alkyl-O-(O)C-C₁₋₆-alkyl, H₂N-C(O)-C₁₋₁₂-Alkyl, C₁₋₆-Alkyl-HN-C(O)-C₁₋₆-alkyl, (C₁₋₆-Alkyl)₂-N-C(O)-C₁₋₆-alkyl; gesättigtes, ungesättigtes oder teilweise gesättigtes C₁₋₁₂-Heterocyclyl, das über ein Kohlenstoffatom gebunden ist und gegebenenfalls ein- oder mehrfach durch C₁₋₆-Alkyl, Trifluormethyl, Nitro, Amino, *N-*mono- oder *N*,*N*-di-C₁₋₆-alkyliertes Amino, C₁₋₆-Alkanoyl, C₂₋₆-Alkenyl, C₂₋₆-Alkinyl, C₁₋₆-Alkoxy, C₁₋₆-Alkoxycarbonylamino, C₀₋₆-Alkylcarbonylamino, C₁₋₆-Alkylcarbonyloxy, C₁₋₁₂-Aryl, *N*-mono- oder *N,N-*di-C₁₋₆-alkyliertes Carbamoyl, gegebenenfalls verestertes Carboxyl, Cyano, Halogen, Halogen-C₁₋₆-alkoxy, Halogen-C₁₋₆-alkyl, C₁₋₁₂-Heteroaryl, gesättigtes, ungesättigtes oder teilweise gesättigtes C₁₋₆-Heterocyclyl, Hydroxyl, Nitro substituiert ist, steht
und R₆ für H oder gegebenenfalls substituiertes C₁₋₁₂-Alkyl, gegebenenfalls substituiertes C₁₋₁₂-Alkylaryl oder gegebenenfalls substituiertes C₁₋₁₂-Aryl steht;
R₇ für H oder eine 0-Schutzgruppe steht;
und zwei beliebige der Gruppen R₇, R₈ oder X gegebenenfalls miteinander zu einer Ringstruktur verknüpft sind;
R₈ für H; gegebenenfalls substituiertes C₁₋₁₂-Alkyl; gegebenenfalls substituiertes C₁₋₁₂-Alkylaryl oder gegebenenfalls substituiertes C₁₋₁₂-Aryl; gegebenenfalls substituiertes C(O)-C₁₋₆-Alkyl; gegebenenfalls substituiertes C(O)O-C₁₋₆-Alkyl; gegebenenfalls substituiertes C(O)NH-C₁₋₆-Alkyl oder gegebenenfalls substituiertes C(O)N(C₁₋₆-Alkyl)₂ steht oder R₈ für
-NHR₉,
-S(O)₂R₉; SOR₉; S(O)₃R₉; S(O)₂N(R₉) ;
-P(O)(R₉)₂
steht oder R₈ für (R₉)₂Y steht, wobei Y für ein Anion wie Acetat oder Halogen steht;
und wobei R₉ jeweils unabhängig voneinander für H, gegebenenfalls substituiertes C₁₋₁₂-Alkyl,
gegebenenfalls substituiertes C₁₋₁₂-Alkylaryl oder gegebenenfalls substituiertes C₁₋₁₂-Aryl steht;
bei dem man
a) eine Verbindung gemäß Formel (11) oder ihre ringgeschlossene Form gemäß Formel (4) oder ein Gemisch davon, 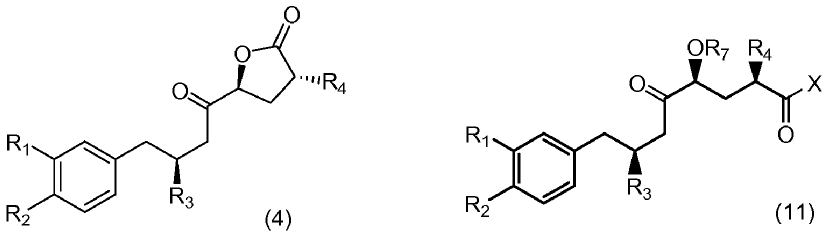 worin R₁, R₂, R₃, R₄, R₇ und X die oben für Formel (2) und (13) angegebene Bedeutung besitzen,
mit einer Verbindung gemäß Formel (5)
R₈-NH₂ (5),
worin R₈ die oben für Formel (2) und (13) angegebene Bedeutung besitzt,
umsetzt, wobei man eine Verbindung gemäß Formel (7) oder eine Verbindung gemäß Formel (12) oder ein Gemisch davon, 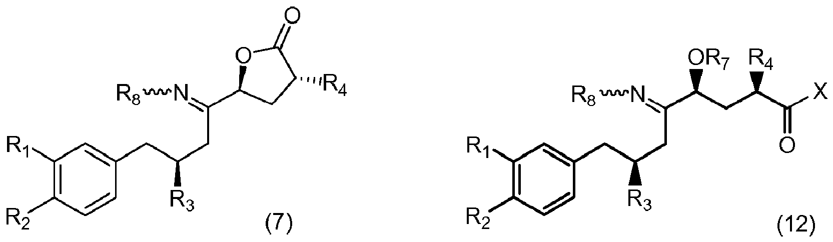 worin R₁, R₂, R₃, R₄, R₇, R₈ und X die oben für Formel (2) und (13) angegebene Bedeutung besitzen,
erhält;
b) weiterhin die Verbindung gemäß Formel (7) oder (12) oder ein Gemisch davon in Gegenwart eines Reduktionsmittels und gegebenenfalls in Gegenwart eines Katalysators und gegebenenfalls in Gegenwart eines oder mehrerer Additive umsetzt,
wobei man eine Verbindung gemäß Formel (2) oder Formel (13) oder ein Gemisch davon erhält.

2. Verfahren nach Anspruch 1, bei dem man die Verbindungen gemäß Formel (7) oder Formel (12) vor der Durchführung von Schritt b) nicht aus der Reaktionsmischung isoliert.

3. Verfahren nach einem der Ansprüche 1-2, bei dem man Schritt b) in Gegenwart eines Katalysators durchführt und es sich bei dem Katalysator um einen auf Übergangsmetall basierenden Katalysator handelt.

4. Verfahren nach einem der Ansprüche 1-3, bei dem man die Verbindung gemäß Formel (11) oder ihre ringgeschlossene Form gemäß Formel (4) oder das Gemisch davon in Gegenwart eines Reduktionsmittels und eines Katalysators mit einer Verbindung gemäß Formel (5) umsetzt.

5. Verfahren nach Anspruch 4, bei dem man das Reduktionsmittel aus der Gruppe bestehend aus molekularem Wasserstoff oder einer wasserstoffspendenden Verbindung auswählt und bei Verwendung des molekularen Wasserstoffs diesen in Gegenwart eines Übergangsmetallkatalysators verwendet und bei Verwendung einer wasserstoffspendenden Verbindung diese in Gegenwart eines Katalysators verwendet.

6. Verfahren nach Anspruch 4 oder 5, bei dem es sich bei dem Katalysator um ein Enzym, vorzugsweise eine Aminotransferase, handelt.

7. Verfahren nach einem der Ansprüche 4-6, bei dem es sich bei der Verbindung gemäß Formel (5) auch um das Reduktionsmittel handelt.

8. Verfahren nach einem der Ansprüche 1-7, bei dem X in der Verbindung gemäß Formel (11) und auch in resultierenden Verbindungen gemäß Formel (12), sofern sie gebildet wird, bzw. der Verbindung gemäß Formel (13) für OR₆ steht und R₆ wie in Anspruch 1 beschrieben ist.

9. Verfahren nach einem der Ansprüche 1-7, bei dem X in der Verbindung gemäß Formel (11) und auch in resultierenden Verbindungen gemäß Formel (12), sofern sie gebildet wird, bzw. der Verbindung gemäß Formel (13) für NHR₅ steht und R₅ wie in Anspruch 1 beschrieben ist.

10. Verfahren zur Herstellung einer Verbindung gemäß Formel (13) oder eines pharmazeutisch unbedenklichen Salzes davon, worin X für NHR₅ steht, wobei das Verfahren ein Verfahren nach einem der Ansprüche 1-9 umfasst und R₅ wie in Anspruch 1 beschrieben ist.

11. Verbindung gemäß Formel (11) 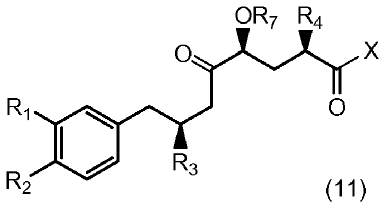 worin alle R-Gruppen die in Anspruch 1 angegebene Bedeutung besitzen und X aus der Gruppe bestehend aus OR₆ und NHR₅ ausgewählt sind und R₆ und R₅ wie in Anspruch 1 beschrieben sind.

12. Verbindung gemäß Formel (12) 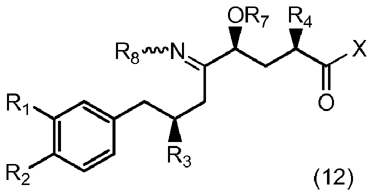 worin alle R-Gruppen die in Anspruch 1 angegebene Bedeutung besitzen und X aus der Gruppe bestehend aus OR₆ und NHR₅ ausgewählt ist und R₆ und R₅ wie in Anspruch 1 beschrieben sind.

13. Verbindung gemäß Formel (7) 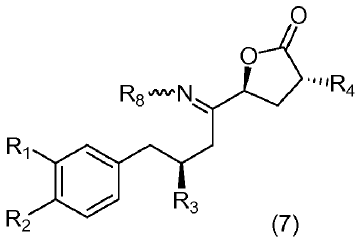 worin R₁, R₂, R₃ und R₄ die in Anspruch 1 angegebene Bedeutung besitzen und R₈ für H; gegebenenfalls substituiertes C₁₋₁₂-Alkyl, gegebenenfalls substituiertes C₁₋₁₂-Alkylaryl oder gegebenenfalls substituiertes C₁₋₁₂-Aryl; gegebenenfalls substituiertes C(O)-C₁₋₆-Alkyl; gegebenenfalls substituiertes C(O)O-C₁₋₆-Alkyl; gegebenenfalls substituiertes C(O)NH-C₁₋₆-Alkyl oder gegebenenfalls substituiertes C(O)N(C₁₋₆-Alkyl)₂ steht oder R₈ für
-NHR₉,
-S(O)₂R₉; SOR₉; S(O)₃R₉; S(O)₂N(R₉);
-P(O)(R₉)₂
steht oder R₈ für (R₉)₂Y steht, wobei Y für ein Anion wie Acetat oder Halogen steht;
und wobei R₉ jeweils unabhängig voneinander für H, gegebenenfalls substituiertes C₁₋₁₂-Alkyl, gegebenenfalls substituiertes C₁₋₁₂-Alkylaryl oder gegebenenfalls substituiertes C₁₋₁₂-Aryl steht.

14. Verfahren nach einem der Ansprüche 1-10, bei dem man die Verbindung gemäß Formel (2) weiterhin mit 3-Amino-2,2-dimethylpropanamid zu einer Verbindung gemäß Formel (13) mit X = NHR₅ oder pharmazeutisch unbedenklichen Salzen davon umsetzt.

15. Verfahren nach einem der Ansprüche 1-10 und 14, bei dem man die Verbindung gemäß Formel (2) oder Formel (13) weiterhin zu 2(S),4(S),5(S),7(S)-N-(2-Carbamoyl-2-methylpropyl)-5-amino-4-hydroxy-2,7-diisopropyl-8-[4-methoxy-3-(3-methoxypropoxy)-phenyl]octanamid oder einem pharmazeutisch unbedenklichen Salz davon umsetzt.

## Revendications

1. Procédé de préparation d'un composé selon la formule (13) ou de sa forme à cycle fermé selon la formule (2), ou d'un mélange de ceux-ci, 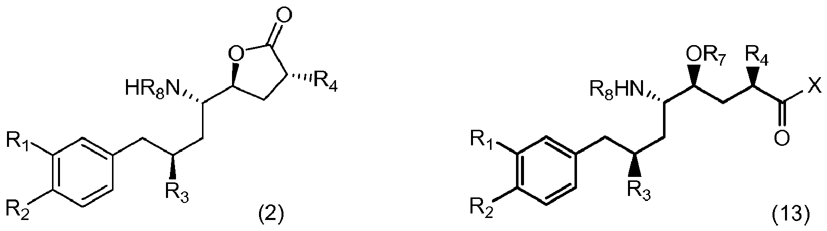 où
R₁ est choisi dans le groupe constitué par F, Cl, Br, I, C₁₋₆halogénoalkyle, C₁₋₆alcoxy, C₁₋₆alcoxy-C₁₋₆alkyloxy, et C₁₋₆alcoxy-C₁₋₆alkyle ;
R₂ est choisi dans le groupe constitué par F, Cl, Br, I, C₁₋₄alkyle ou C₁₋₄alcoxy ;
R₁ et R₂ peuvent être liés ensemble pour former une structure cyclique ;
R₃ et R₄ sont chacun indépendamment C₃₋₆alkyle ramifié ; X représente NHR₅ ou OR₆ où
R₅ est C₁₋₁₂cycloalkyle, C₁₋₁₂alkyle, C₁₋₁₂hydroxyalkyle, C₁₋₆alcoxy-C₁₋₆alkyle, C₁₋₆alcanoyloxy-C₁₋₆alkyle, C₁₋₁₂-aminoalkyle, C₁₋₆alkylamino-C₁₋₆alkyle, C₁₋₆dialkylamino-C₁₋₆alkyle, C₁₋₆alcanoylamino-C₁₋₆alkyle, HO-(O)C-C₁₋₁₂-alkyle, C₁₋₆alkyl-O-(O)C-C₁₋₆alkyle, H₂N-C(O)-C₁₋₁₂alkyle, C₁₋₆alkyl-HN-C(O)-C₁₋₆alkyle, (C₁₋₆alkyl)₂-N-C(O)-C₁₋₆alkyle ; C₁₋₁₂hétérocyclyle saturé, insaturé ou partiellement saturé lié par l'intermédiaire d'un atome de carbone, et lequel hétérocyclyle est éventuellement substitué une ou plusieurs fois par C₁₋₆alkyle, trifluorométhyle, nitro, amino, N-mono- ou N,N-di-C₁₋₆alkylamino, C₁₋₆alcanoyle, C₂₋₆alcényle, C₂₋₆alcynyle, C₁₋₆alcoxy, C₁₋₆alcoxycarbonylamino, C₀₋₆alkylcarbonylamino, C₁₋₆alkylcarbonyloxy, C₁₋₁₂aryle, N-mono ou N,N-di-C₁₋₆alkylcarbamoyle, carboxyle éventuellement estérifié, cyano, halogène, halogéno-C₁₋₆alcoxy, halogéno-C₁₋₆-alkyle, C₁₋₁₂hétéroaryle, C₁₋₆hétérocyclyle saturé, insaturé ou partiellement saturé, hydroxyle, nitro ; et R₆ représente H, ou C₁₋₁₂alkyle éventuellement substitué, C₁₋₁₂alkylaryle éventuellement substitué, ou C₁₋₁₂aryle éventuellement substitué ;
R₇ représente H, ou est un groupement protecteur de O ; et où deux quelconques parmi R₇, R₈, ou X sont éventuellement liés ensemble pour former une structure cyclique ;
R₈ désigne H ; C₁₋₁₂alkyle éventuellement substitué, C₁₋₁₂alkylaryle éventuellement substitué, ou C₁₋₁₂aryle éventuellement substitué ; C(O)C₁₋₆alkyle éventuellement substitué ; C(O)OC₁₋₆alkyle éventuellement substitué ; C(O)NHC₁₋₆alkyle éventuellement substitué ; ou C(O)N(C₁₋₆alkyl)₂ éventuellement substitué ;
ou R₈ désigne -NHR₉, -S(O)₂R₉ ; SOR₉ ; S(O)₃R₉ ; S(O)₂N(R₉) ; -P(O)(R₉)₂ OU
R₈ représente (R₉)₂Y, Y étant un anion tel qu'acétate, ou halogène ; et où chaque R₉ représente indépendamment H, C₁₋₁₂alkyle éventuellement substitué, C₁₋₁₂alkylaryle éventuellement substitué, ou C₁₋₁₂aryle éventuellement substitué ;
comprenant les étapes suivantes consistant à
a) réagir un composé selon la formule (11), ou sa forme à cycle fermé selon la formule (4), ou un mélange de ceux-ci, 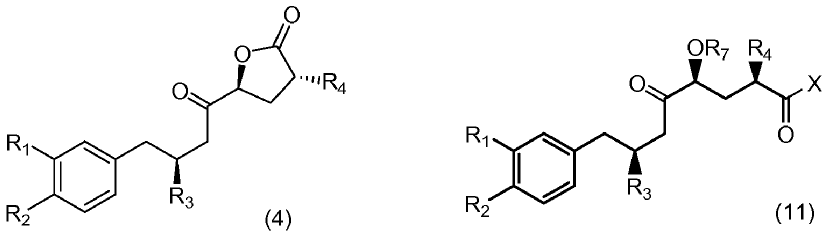 où R₁, R₂, R₃, R₄, R₇ et X sont chacun tels que décrits ci-dessus pour la formule (2) et (13)
avec un composé selon la formule (5)
R₈-NH₂ (5)
où R₈ est tel que décrit ci-dessus pour la formule (2) et (13)
laquelle réaction conduit à un composé selon la formule (7) ou un composé selon la formule (12) ou un mélange de ceux-ci, 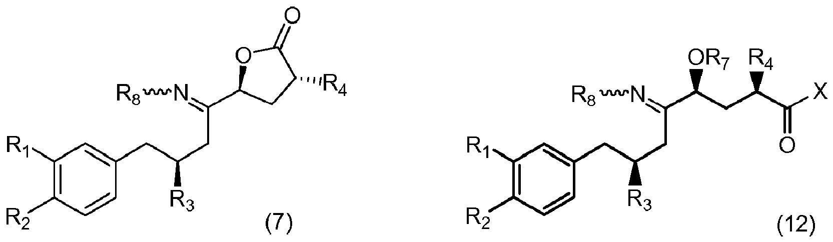 où R₁, R₂, R₃, R₄, R₇, R₈ et X sont chacun tels que décrits ci-dessus pour la formule (2) et (13)
b) réagir davantage un composé selon la formule (7) ou (12) ou un mélange de ceux-ci, en présence d'un réactif réducteur, et éventuellement en présence d'un catalyseur, et éventuellement en présence d'un ou plusieurs additifs,
laquelle réaction conduit à la formation du composé selon la formule (2) ou formule (13) ou d'un mélange de ceux-ci.

2. Procédé selon la revendication 1, dans lequel les composés selon la formule (7) ou formule (12) ne sont pas isolés du mélange réactionnel avant la réalisation de l'étape b).

3. Procédé selon l'une quelconque des revendications 1-2, dans lequel l'étape b) est réalisée en présence d'un catalyseur et où ledit catalyseur est un catalyseur à base d'un métal de transition.

4. Procédé selon l'une quelconque des revendications 1-3, comprenant la réaction du composé selon la formule (11), ou de sa forme à cycle fermé selon la formule (4), ou d'un mélange de ceux-ci, avec un composé selon la formule (5) en présence d'un agent réducteur et d'un catalyseur.

5. Procédé selon la revendication 4, dans lequel l'agent réducteur est choisi dans le groupe constitué par l'hydrogène moléculaire ou un composé donneur d'hydrogène ; et où lorsque l'hydrogène moléculaire est utilisé, il est utilisé en présence d'un catalyseur à base d'un métal de transition et lorsqu'un composé donneur d'hydrogène est utilisé, il est utilisé éventuellement en présence d'un catalyseur.

6. Procédé selon la revendication 4 ou 5, dans lequel ledit catalyseur est une enzyme, préférablement une aminotransférase.

7. Procédé selon l'une quelconque des revendications 4-6, dans lequel le composé selon la formule (5) est également l'agent réducteur.

8. Procédé selon l'une quelconque des revendications 1-7, dans lequel X dans le composé selon la formule (11), et aussi dans les composés résultants selon la formule (12), s'il est formé, et le composé selon la formule (13), respectivement, représente OR₆ et où R₆ est tel que décrit selon la revendication 1.

9. Procédé selon l'une quelconque des revendications 1-7, dans lequel X dans le composé selon la formule (11), et aussi dans les composés résultants selon la formule (12), s'il est formé, et le composé selon la formule (13), respectivement, représente NHR₅, et où R₅ est tel que décrit selon la revendication 1.

10. Procédé de préparation d'un composé selon la formule (13) ou d'un sel quelconque pharmaceutiquement acceptable de celui-ci, où X représente NHR₅, où le procédé comprend un procédé selon l'une quelconque des revendications 1-9 et où R₅ est tel que décrit selon la revendication 1.

11. Composé selon la formule (11) 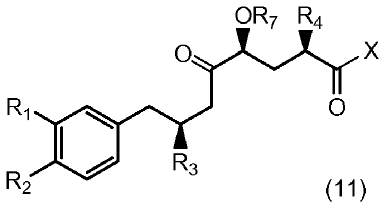 dans laquelle tous les groupements R sont tels que définis selon la revendication 1, et où X est choisi dans le groupe constitué par OR₆ et NHR₅, et où R₆ et R₅ sont tels que décrits selon la revendication 1.

12. Composé selon la formule (12) 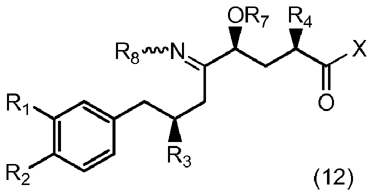 dans laquelle tous les groupements R sont tels que définis selon la revendication 1 et où X est choisi dans le groupe constitué par OR₆ et NHR₅, et où R₆ et R₅ sont tels que décrits selon la revendication 1.

13. Composé selon la formule (7) 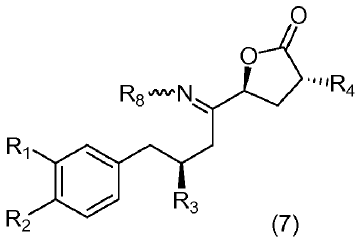
dans laquelle R₁, R₂, R₃, et R₄, sont tels que définis selon la revendication 1, et où R₈ désigne H ; C₁₋₁₂-alkyle éventuellement substitué, C₁₋₁₂alkylaryle éventuellement substitué, ou C₁₋₁₂aryle éventuellement substitué ; C(O)C₁₋₆alkyle éventuellement substitué ; C(O)OC₁₋₆alkyle éventuellement substitué ;
C(O)NHC₁₋₆alkyle éventuellement substitué ; ou C(O)N(C₁₋₆alkyl)₂ éventuellement substitué ;
ou R₈ désigne -NHR₉, -S(O)₂R₉ ; SOR₉ ; S(O)₃R₉ ; S(O)₂N(R₉) ; -P(O)(R₉)₂ ;
ou R₈ représente (R₉)₂Y, Y étant un anion tel qu'acétate, ou halogène ;
et où chaque R₉ représente indépendamment H, C₁₋₁₂alkyle éventuellement substitué, C₁₋₁₂alkylaryle éventuellement substitué, ou C₁₋₁₂aryle éventuellement substitué.

14. Procédé selon l'une quelconque des revendications 1-10, dans lequel le composé selon la formule (2) est réagi davantage avec du 3-amino-2,2-diméthylpropanamide afin d'obtenir un composé selon la formule (13) avec X=NHR₅, ou des sels pharmaceutiquement acceptables de celui-ci.

15. Procédé selon l'une quelconque des revendications 1-10 et 14, dans lequel le composé selon la formule (2) ou la formule (13) est réagi davantage afin d'obtenir le 2(S),4(S),5(S),7(S)-N-(2-carbamoyl-2-méthylpropyl)-5-amino-4-hydroxy-2,7-diisopropyl-8-[4-méthoxy-3-(3-méthoxypropoxy)phényl]octanamide ou un sel quelconque pharmaceutiquement acceptable de celui-ci.
